(19) Europäisches Patentamt
European Patent Office
Office européen des brevets

(11) Publication number : **0 592 306 A2**

(12) **EUROPEAN PATENT APPLICATION**

(21) Application number : **93402454.8**

(51) Int. Cl.$^5$ : **A61K 49/00**

(22) Date of filing : **06.10.93**

(30) Priority : **06.10.92 JP 267669/92**
**09.09.93 JP 224571/93**

(43) Date of publication of application :
**13.04.94 Bulletin 94/15**

(84) Designated Contracting States :
**AT BE CH DE DK ES FR GB GR IE IT LI LU MC NL PT SE**

(71) Applicant : **TERUMO Kabushiki Kaisha**
**44-1 Hatagaya 2-chome Shibuya-ku**
**Tokyo (JP)**

(72) Inventor : **Kawanishi, Tetsuro**
**2-6-29, Isehara, Isehara-shi,**
**Kanagawa-Ken (JP)**
Inventor : **Asai, Hiroyuki**
**9-41, Izumi-cho**
**Mishima-shi, Shizuoka-Ken (JP)**

(74) Representative : **Gillard, Marie-Louise et al**
**Cabinet Beau de Loménie 158, rue de l'Université**
**F-75340 Paris Cédex 07 (FR)**

(54) **19F-MRI Contrast medium.**

(57) A $^{19}$F-MRI contrast medium for MRI using $^{19}$F as a detectable nucleus, comprising a metal complex compound in which a polyamine ligand containing not less than one fluorine atom is coordinate-bonded to an ion of a paramagnetic metal. Also disclosed is a specific polyamine metal complex compound suitable for the $^{19}$F-MRI contrast medium.

EP 0 592 306 A2

The present invention relates to an MRI contrast medium and, more particularly, to an MRI contrast medium using fluorine as a detectable nucleus.

Magnetic resonance imaging (to be referred to as MRI hereinafter) is one of imaging diagnoses, like X-ray imaging, ultrasonic imaging, and nuclear medicine imaging. These imaging diagnoses can visually image pathological changes of living bodies. The imaging diagnoses are, therefore, very excellent means for making accurate diagnoses of diseases and are already used extensively. Among other imaging diagnoses, MRI is a promising imaging diagnosis which has been rapidly spread and developed in recent years.

MRI which is currently being employed in clinical examinations uses $^{1}H$ as a detectable nucleus. A measurement target in such MRI is primarily water molecules which exist in a large amount in a living tissue. The principle of this $^{1}H$-MRI is as follows.

The relaxation time, the $^{1}H$ density, the $^{1}H$ chemical shift, and the like of $^{1}H$ constituting a water molecule change depending on an environment in which the water molecule is present. In particular, water molecules present in different tissues of a living body can be distinguished from each other by the difference in relaxation time of $^{1}H$ between the water molecules. Therefore, by measuring the relaxation time of $^{1}H$ for many water molecules distributed in a living body and imaging the differences in relaxation time between these water molecules, various tissues of the living body can be imaged. Likewise, it is also possible to distinguish a water molecule in a pathologically abnormal tissue from a water molecule in a normal tissue in accordance with the difference in relaxation time of $^{1}H$ between these water molecules, and so the pathologically abnormal tissue shows an MRI image different from that of the normal tissue. Therefore, pathological abnormalities can be diagnosed on the basis of MRI images.

Recently, on the other hand, MRI diagnoses using nuclides other than $^{1}H$ as detectable nuclei are also being attempted. Examples of the nuclide other than $^{1}H$, which are NMR-spectroscopically detectable, are $^{19}F$, $^{23}Na$, $^{31}P$, and $^{13}C$. When the relative sensitivity and the isotope abundance are taken into account, however, nuclides practically applicable to the MRI diagnoses are $^{19}F$ and $^{31}P$. These two nuclides have already been researched for clinical applications. An example of the applications examined is MRS (magnetic resonance spectroscopy) using $^{31}P$ as a detectable nucleus to observe the distribution of ATP, ADP, creatine phosphate, inorganic phosphoric acid, and the like in a living body and use the observation result in diagnoses. Since, however, the sensitivity of $^{31}P$ is low, 6% that of $^{1}H$, $^{31}P$ has its measurement limit, and this makes imaging using $^{31}P$ difficult. Therefore, clinical applications of $^{31}P$ are also limited.

$^{19}F$, in contrast, has the following characteristics and hence is considered as a nuclide with the highest possibility of being applied to clinical examinations.

(1) $^{19}F$ has a high sensitivity, 83% that of $^{1}H$.

(2) Since the resonance frequency of $^{19}F$ is close to that of $^{1}H$, measurements can be performed by using an MRI apparatus for $^{1}H$.

(3) $^{19}F$ is an inexpensive element with a natural abundance of 100%.

(4) $^{19}F$ does not exist in any living tissues but teeth. This makes it possible to perform imaging diagnoses using $^{19}F$ as a tracer by using a fluorine-containing compound as a contrast medium.

(5) It is also possible to obtain functional information, such as the biochemical environment and the metabolic state of a living tissue, from a change in biochemical the chemical shift of $^{19}F$. This purpose may be accomplished by using a contrast medium compound containing $^{19}F$, the chemical structure of which changes in accordance with changes in the biochemical environment and metabolic state of a living body, and which consequently changes the chemical environment surrounding $^{19}F$.

As described above, $^{19}F$-MRI has characteristics entirely different from those of the conventional $^{1}H$-MRI and can obtain new information useful in diagnoses. The MRI using $^{19}F$ therefore has an extremely high utility value.

For example, when a fluorine compound having compatibility with blood is used as a contrast medium, a portion where a blood flow is present can be selectively imaged. This selective imaging of a blood flow is applicable to identification of an ischemia portion or a necrosis tissue. In addition, selective imaging of a particular tissue is possible when a substance which specifically recognizes a particular organ, a particular lesion, or a particular receptor is labeled with fluorine and used as a contrast medium.

Researches for applying the MRI diagnosis using $^{19}F$ as a detectable nucleus to clinical examinations on the basis of the above potential utility value have already been reported or disclosed. Some of these researches are exemplified below.

(a) Attempts to image blood vessels or organs by administering, as a contrast medium, perfluorocarbon which has been used for artificial blood [Investigative Radiology, 20, 504 - 509 (1985); Investigative Radiology, 23, S298 - S301 (1988); Journal of Computer Assisted Tomography, 9(1), 8 - 15 (1985)].

(b) Imaging of the difference between oxygen concentrations in a living body as contrast by using perfluorotripropylamine as a contrast medium [Magnetic Resonance Imaging, 5, 279 - 285 (1987)].

(c) Imaging of the brains of rats by using well-known 2-fluoro-2-deoxy-D-glucose as a contrast medium in PET (Positron Emission Tomography) [Magnetic Resonance Imaging, 6, 633 - 635 (1988)].

(d) Imaging using 5-fluorouracil which is a known anticancer agent [NMR in Biomedicine, 1 (No. 3), 113 - 120 (1988)].

(e) A method of imaging the pH distribution in a living tissue as the difference in the chemical shift of $^{19}F$ by using a fluorine-substituted benzene derivative [USP. No. 5,130,119.

(f) A method of specifically imaging a tissue, such as a cancer lesion, by using an antibody modified with 100 or more fluorine atoms [Jpn. Pat. Appln. KOKAI Publication No. 63-135337].

In addition to the above examples, a large number of attempts to clinically apply the MRI diagnosis using $^{19}F$ as a detectable nucleus have been reported. However, the $^{19}F$-MRI has not spread in clinical diagnoses yet. The primary cause for this is assumed to be the insufficient detection sensitivity of a fluorine compound administered as a contrast medium.

That is, since the detection sensitivity of a contrast medium is insufficient, a high-magnetic-field MRI apparatus which has not been clinically used must be employed in order to obtain enough information for diagnoses. An extremely long imaging time, on the other hand, is required in attempting to obtain enough information by using a clinical MRI apparatus that is normally used instead of the high-magnetic-field MRI apparatus. These situations prevent a wide spread of the $^{19}F$-MRI in clinical examinations.

The use of a large quantity of a fluorine compound may increase the imaging sensitivity. In this case, however, the toxicity of that fluorine compound poses a problem. As an example, in the blood vessel imaging experiment (report example (a) described above) using perfluorocarbon as a contrast medium, it is reported that a large amount of the contrast medium is necessary to obtain high-quality images. An application of such the imaging method to human bodies accompanies a serious problem in respect of toxicity.

In addition, when tissue specific imaging is performed by using, as a contrast medium, a substance which has a specific tissue affinity (e.g., a monoclonal antibody; to be referred as a tissue specific substance hereinafter) modified with fluorine, the contrast with respect to the background is impaired by the use of a large amount of the contrast medium. On the other hand, it is practically impossible to improve the detection sensitivity by modifying this tissue specific substance with a large number of fluorine atoms for the reasons to be explained below. First, it is very difficult in view of the synthesis reaction to perform modification of the substance with such a large number of fluorine atoms. Second, even if the modification is possible, this modification with such a large number of fluorine atoms degrades the tissue specificity of the tissue specific substance and also leads to an increase in toxicity.

It is the first object of the present invention to provide a $^{19}F$-MRI contrast medium for MRI using $^{19}F$ as a detectable nucleus, which has a satisfactory imaging performance within the range of a contrast medium concentration that is physiologically acceptable, and which makes it possible to obtain image information useful in diagnoses even when an MRI apparatus that has been normally used in clinical MRI diagnoses is employed.

It is the second object of the present invention to provide a metal complex compound with fluorine atoms, which is useful as the above $^{19}F$-MRI contrast medium.

The above first object of the present invention is achieved by a contrast medium for MRI using $^{19}F$ as a detectable nucleus, comprising a metal complex compound in which a polyamine ligand containing one or more fluorine atoms is coordinate-bonded to a paramagnetic metal ion.

The above second object of the present invention is achieved by a metal complex compound represented by the following formula and its physiologically acceptable salts :

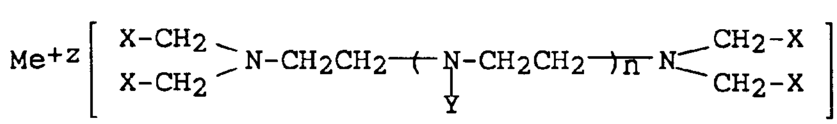

wherein Me, z, X, n, and Y have the following meanings;

Me : paramagnetic metal ion

z : the ionic valence of Me, a positive integer (preferably 2 or 3)

X : which may be the same or different and represents $-COO^-$, -COOZ, $-PO_3HZ$, -CONHW, or -OH wherein Z and W are;

Z : a hydrogen atom, an organic or inorganic base equivalent or metal ion equivalent

W : a straight chain or branched chain alkyl group substituted with not less than one -OH group

n : an integer from 1 to 3

Y : when n = 1, Y is R, wherein R is a substituent which has not less than one fluorine atom and may contain X defined above, and

when n = 2 or 3, at least one Y is R defined above and each of remaining Ys is $-CH_2X$, a lower alkyl group, or a hydrogen atom.

and wherein a ligand portion of the metal complex compound is coordinate-bonded to the paramagnetic metal ion via at least some of nitrogen atoms and/or oxygen atoms which are contained in the ligand and can be coordinated.

The principal characteristic feature of the $^{19}F$-MRI contrast medium of the present invention is to use, as a compound having imaging activity, a polyamine metal complex compound in which a polyamine ligand containing $^{19}F$ is coordinate-bonded to a paramagnetic metal ion.

In the $^{19}F$-MRI contrast medium of the present invention, the paramagnetic metal ion is not particularly limited so far as it has paramagnetism and forms a stable complex. Examples of the paramagnetic metal ion are divalent and trivalent ions of transition metals with atomic numbers 21 to 29, 42, and 44 as well as lanthanoid-series metals with atomic numbers 58 to 70. Of these ions, particularly preferred are ions of chromium, manganese, iron, copper, and gadolinium having stronger paramagnetism.

In the $^{19}F$-MRI contrast medium of the present invention, the polyamine ligand is an organic ligand containing a plurality of amine nitrogen atoms which can be coordinated to the paramagnetic metal ion. A fluorine atom contained in the polyamine ligand is the one that has a $^{19}F$ nucleus, and it is preferable that a plurality of these fluorine atoms be contained in the ligand. Preferably, the NMR chemical shifts of these fluorine atoms are essentially the same. "The chemical shifts are essentially the same" means that the chemical shift values of all fluorine atoms are distributed within a sufficiently narrow range (preferably within a range of 2,000 Hz) so that signals from all the fluorine atoms are effectively sampled in MRI measurement and no artifact images are formed.

In the $^{19}F$-MRI contrast medium of the present invention, it is desirable that a tissue specific substance having a specific affinity for a particular living tissue to be imaged is bonded to the polyamine ligand.

The $^{19}F$-MRI contrast medium of the present invention is desirably prepared into tablets, a powdered medicine, a liquid medicine, and the like according to a given prescription by employing conventional preparation techniques so that the medium is suitably admistered to living bodies. In the preparation, it is possible to use assistants or additives that are commonly used in the preparation techniques, such as fillers, stabilizers, surfactants, buffering agents, electrolytes, coloring agents, perfumes, and flavoring agents.

The $^{19}F$-MRI contrast medium of the present invention has the following advantages.

The first advantage is to be able to significantly amplify the signal intensity in MRI measurement because $^{19}F$ and the paramagnetic metal ion exist in the same molecule. This advantage results from the fact that the relaxation time of $^{19}F$ shortens under the effect of the paramagnetic metal. The details of the first advantage will be described below.

The magnetic properties of the paramagnetic metal have an effect on the nuclear spin of $^{19}F$ in the vicinity of the metal, shortening the $T_1$ value (longitudinal relaxation time) and the $T_2$ value (transverse relaxation time) in $^{19}F$-NMR. The closer the paramagnetic metal and $^{19}F$, the stronger the effect. In the present invention, since the paramagnetic metal and $^{19}F$ exist in the same molecule and are therefore very close to each other, the effect of shortening the relaxation time is extremely large. As a result, in the contrast medium of the present invention, it is possible to obtain very small $T_1$ and $T_2$ values, several milliseconds to several tens of milliseconds, in an aqueous 10 mM solution. Since the $T_1$ and $T_2$ values of a general fluorine compound, such as perfluorocarbon, are on the order of seconds, the relaxation time of $^{19}F$ in the contrast medium of the present invention can be largely shortened to 1/100 to 1/1,000 under the effect of the paramagnetic metal. Note that the relaxation time of the contrast medium of the present invention remains constant regardless of the concentration of the medium because the paramagnetic metal and $^{19}F$ exist in the same molecule. Since the relaxation time is largely shortened as described above, a significantly large signal intensity amplification shown below can be obtained in a pulse sequence of MRI.

When a spin echo method that is clinically most common is used as a pulse sequence method, a signal intensity per pulse is given by the following relation:

$$\text{signal intensity} \propto \text{F atomic density} \cdot \exp(-TE/T_2) \cdot [1 - \exp(-TR/T_1)]$$

where TE is the echo time and TR is the repetition time.

As is apparent from the above relation, the signal intensity per pulse increases as $T_1$ decreases. Therefore, a significantly high signal intensity per pulse can be obtained when the relaxation time ($T_1$ and $T_2$ values) is largely shortened as described above. In addition, since a short repetition time (TR) of pulse radiation can be set when $T_1$ is shortened, the number of signals that can be extracted in a given unit time increases. This also makes it possible to largely amplify the intensity of a finally extracted signal by integrating a larger number of signals.

In the case of a spin echo method using common imaging conditions, a shorter $T_1$ is more advantageous as described above. In contrast, a shorter $T_2$ is more disadvantageous because the signal intensity in MRI

measurement decreases if $T_2$ shortens. As described above, the paramagnetic metal has the effect of shortening the relaxation time substantially evenly on $T_1$ and $T_2$, and so $T_2$ is also shortened significantly in the contrast medium of the present invention. This disadvantage caused by the short $T_2$, however, can be avoided by controlling the imaging conditions. That is, by adopting proper imaging parameters, only the advantage obtained by the short $T_1$ appears significantly, making it possible to perform MRI measurement that does not easily suffer from the disadvantage caused by the short $T_2$. More specifically, the above desirable outcome can be obtained by setting a value of the echo time (TE) as short as possible (preferably a value shorter than $T_2$) and setting the repetition time (TR) at about 1.5 times the value of $T_1$.

Since, however, the setting of the imaging conditions of an MRI apparatus has its limit, the imaging conditions for obtaining the desirable result as described above cannot be adopted in some cases depending on the apparatus and the contrast medium to be used. Especially, in case of using an MRI apparatus having lower limits of TE and TR, it may be impossible to perform the desirable MRI imaging as described above when a contrast medium has an extremely short relaxation time ($T_1$ and $T_2$). Even in such a case, MRI under the above desirable imaging conditions can be performed by using a contrast medium having a longer relaxation time within a range in which the imaging sensitivity does not decrease. It is also an advantage of the present invention to be able to control the relaxation time of a contrast medium which is to be used in this manner. This control of the relaxation time is achieved by molecular design of the polyamine metal complex compound contained in the contrast medium of the present invention. In the molecular design for controlling the relaxation time of $^{19}F$, the first factor is the type of the paramagnetic metal ion, and the second factor is the distance between the paramagnetic metal ion and $^{19}F$.

The first factor is based on the fact that the effect of shortening the relaxation time is varied depending on the type of the paramagnetic metal ion. The relaxation effect of the paramagnetic metal has correlation with the magnitude of its magnetic moment which is proportional to the number of unpaired electrons that the metal ion has. As an example, the magnitude of the magnetic moment has a sequence of $Cd^{3+} > Mn^{2+} > Cr^{3+} > Fe^{3+} > Cu^{2+}$, and the magnitude of the effect of shortening the relaxation time matches this sequence. In the $^{19}F$-MRI contrast medium of the present invention, therefore, the relaxation time can be controlled by selecting the type of the paramagnetic metal ion. That is, the relaxation time can be shortened by selecting a paramagnetic metal with a large magnetic moment and prolonged by selecting a metal with a small magnetic moment.

The second factor is based on the fact that the relaxation effect of the paramagnetic metal ion has correlation with the distance between the paramagnetic metal ion and $^{19}F$. Therefore, the relaxation time can be prolonged by increasing this distance and shortened by decreasing the distance. The distance between the paramagnetic metal ion and $^{19}F$ can be increased easily by inserting an appropriate spacer between the coordination site and the $^{19}F$-introduction site of the polyamine ligand. Such a spacer is not particularly limited, and an alkyl chain or an aromatic ring is usable.

The second advantage of the $^{19}F$-MRI contrast medium according to the present invention is to be able to easily introduce a plurality of fluorine atoms into each molecule of a contrast medium compound. That is, the polyamine ligand has one or more N atoms to which a substituent can be introduced easily. Therefore, a fluoroalkyl group having one or more fluorine atoms can be introduced to these N atoms so that a plurality of fluorine atoms are contained in one entire molecule. Introducing a plurality of fluorine atoms in this manner makes it possible to obtain more intense MRI signals, improving the detection sensitivity of the contrast medium. This is because the intensity of MRI signals in $^{19}F$-MRI is proportional to the density of fluorine atoms, i.e., the number of fluorine atoms contained in the molecule.

The third advantage is that a plurality of fluorine atoms having essentially the same chemical shift can be introduced easily into the molecule of the contrast medium compound. That is, since the metal complex compound containing polyamine as a ligand has a good molecular symmetry, a plurality of substitution positions that are essentially the same in chemical shift environment can exist. By using these substitution positions, a plurality of fluorine atoms having essentially the same chemical shift can be introduced into a molecule. The signal sampling efficiency in MRI measurement can be improved because a plurality of fluorine atoms have essentially the same chemical shift. This also effectively prevents, in MRI imaging, formation of chemical shift articraft images that interfere with diagnoses. In contrast, in a conventional contrast medium having a plurality of fluorine atoms, such as perfluorocarbon, the chemical shifts of these fluorine atoms essentially differ from one another. Therefore, it is impossible that signals from all of the fluorine atoms can be sampled effectively. In addition, articraft images are produced.

The fourth advantage of the $^{19}F$-MRI contrast medium according to the present invention is its extremely low toxicity regardless of the presence of metal ions. This results from the fact that the polyamine ligand is an excellent multidentate ligand, as is well known for EDTA, and hence forms a stable complex with metal ions. Many free metal ions exhibit toxicity by interfering with biochemical reactions in living bodies, such as metabolism. Therefore, an instable complex which frees metal ions in a living body shows toxicity caused by these

free metal ions. In contrast, many polyamine metal complexes used in the contrast medium of the present invention have a high complex formation constant ($logK_{ML}$) of 15 to 25. These values demonstrate that almost no metal ions are freed under the conditions in a living body and hence the metal ions cannot exhibit their toxicity.

The fifth advantage of the $^{19}F$-MRI contrast medium according to the present invention is that the polyamine metal complex as an imaging active substance can be chemically modified easily. Since a compound having no active reaction site, such as perfluorocarbon, is low in reactivity, it is very difficult to chemically modify such a compound through an usual chemical reaction. The polyamine metal complex of the present invention, on the other hand, can be easily subjected to various chemical modifications corresponding to intended applications, by using nitrogen atoms present in the polyamine ligand portion of the complex. It is also possible to introduce a reactive site into a substituent portion having fluorine atoms or a spacer portion, and a given chemical modification can be performed by using this reactive site.

The first objective of such a chemical modification is to impart tissue specificity to the contrast medium of the present invention. For example, organ specificity or lesion specificity can be imparted by controlling the size, the hydrophobic/hydrophilic balance, and the charge of a molecule. A most useful method for this purpose is to form a composite of a polyamine metal complex and a tissue specific substance having a specific affinity for a particular living tissue. As such a tissue specific substance, there are various known substances such as proteins, peptides sugars, glycoproteins, lipids, and other organic compounds. Practical examples of the tissue specific substance are a monoclonal antibody and its fragment for a particular tissue, hormones, a neurotransmitter, a drug which binds to a particular receptor, an organ cumulative substance, and a metabolic substrate compound. These substances may be either substances originating from living bodies or synthetic substances.

To form a composite of a polyamine metal complex and a tissue specific substance, the tissue specific substance is combined with the polyamine ligand via a proper bond, such as an ester bond, an amide bond, an amide bond, or a disulfide bond. For this purpose, a polyamine metal complex in which an appropriate reactive group is introduced to the ligand portion can be reacted with the tissue specific substance. This causes the reactive group introduced to the ligand portion to react with a reactive group (e.g., an amino group, a thiol group, a hydroxyl group, a carboxyl group, or an imidazole group) contained in the tissue specific substance, forming the bond as described above between them. The reactive group to be introduced to the polyamine ligand portion is properly selected from, e.g., an acid anhydride group, an acid halide group, an active ester group, a nitrene group, an isocyanate group, an isothiocyanate group, and a maleimide group in accordance with the reactive group contained in the tissue specific substance. The reactive group to be introduced to the ligand portion is preferably introduced while being protected by a proper protective group, and activated by eliminating the protective group immediately before the reaction with the tissue specific substance. If possible, the tissue specific substance may be bonded to the polyamine metal complex by using a coupling reagent, such as carbodiimide.

Tissue specific imaging is possible by bonding a tissue specific substance to a polyamine metal complex as described above. Examples of a target tissue in this imaging may be a tumor tissue, an infectious tissue, an inflammatory tissue, a necrotic tissue, a metabolic abnormal tissue, a particular metabolite cumulative tissue, and a particular receptor existing tissue. This tissue specific imaging is extremely useful in MRI diagnoses of various diseases, such as cancers, infectious diseases, ischemic heart diseases, arteriosclerosis, ulcers, and mental diseases. The illustrative details of this tissue specific imaging are as follows.

In specific imaging of a tumor tissue, an antibody whose antigen is a tumor tissue is normally used. A large number of researches on antibodies against a tumor tissue have been reported. Applications of these antibodies to external diagnoses and nuclear medicine have been attempted, and the antibodies have already been used in clinical diagnoses. MRI using such an antibody as the tissue specific substance is useful in diagnoses of, e.g., malignant melanoma, carcinoma of the colon and rectum, pancreatic cancer, and malignant lymphoma. In specific imaging of an inflammatory tissue, a substance having affinity for activated leucocytes which appear in a large number in an inflammatory portion can be used as the tissue specific substance. In addition, leucocytes themselves can be used as the tissue specific substance. To specifically image a necrotic tissue of a cardiac muscle, a monoclonal antibody against myosin can be used as the tissue specific substance.

The above tissue specific imaging can also be applied to receptor mapping by using, as the tissue specific substance, agonist, antagonist, hormone, or a chemical transmitter, each of which acts on a particular receptor, or an antibody against the receptor.

For the purpose of the above tissue specific imaging, a tissue specific substance with the broadest application range is an antibody. An antibody used for this purpose preferably has the following properties.

(1) Having specificity for a target tissue and not having cross reactivity for other tissues. In this respect, a monoclonal antibody is particularly desirable.

(2) Acting on an antigen present at a high concentration in a living body and having a high affinity for that

antigen.

(3) Acting on an antigen which exists only in a tissue, such as a cell membrane, and is not freed into blood. Although an antibody may be used in the form of a whole antibody molecule, it can also be used as an antibody fragment containing an antigen recognizing portion. The use of this antibody fragment provides the following merits. First, since the background signal vanishes rapidly, cumulation in a target tissue can be detected within a short time period. This is because the antibody fragment has a short half life in a living body and hence vanishes rapidly except in a target tissue. Second, the antibody fragment hardly decreases its tissue specificity when used continuously compared to a whole antibody molecule. This is so because an antibody against the antibody fragment is more difficult to be produced than an antibody against a whole antibody molecule. The antibody fragment obtained by removing an Fc portion not participating in antigen recognition from a whole antibody molecule are used frequently. Examples are an Fab fragment obtained by subjecting a whole antibody molecule to a papain treatment, and an F(ab') fragment obtained by performing a pepsin treatment of a whole antibody molecule.

The second object of the chemical modification performed for the imaging active substance (i.e., the polyamine metal complex) of the present invention is to detect changes of tissue environments, such as a pH, an oxygen concentration and/or an oxidation-reduction potential, by converting the environmental changes into the chemical shift changes of $^{19}F$. This makes it possible to obtain information concerning tissue environments from the chemical shift of $^{19}F$, resulting that imaging of, e.g., an abnormal metabolic tissue becomes possible. An example of such a chemical modification is to introduce a ring structure, which closes or opens depending on the change in pH, into a ligand molecule in the vicinity of $^{19}F$.

As described above, the $^{19}F$-MRI contrast medium of the present invention has a much higher sensitivity and a lower toxicity than those of conventional $^{19}F$-MRI contrast media, such as perfluorocarbon. Therefore, the use of the $^{19}F$-MRI contrast medium of the present invention makes it possible to realize clinical applications of $^{19}F$-MRI, resulting that MRI diagnoses for various purposes entirely different from those of $^{1}H$-MRI can be made.

For example, the $^{19}F$-MRI contrast medium of the present invention is applicable to image diagnoses which is currently made by means of nuclear medicine, such as blood flow scintigraphy using radioisotopes. That is, the presence of a blood flow can be imaged by administering the $^{19}F$-MRI contrast medium (of a water-soluble type) of the present invention into blood and imaging using the medium as a tracer. Like the blood flow scintigraphy, such an MRI diagnosis is useful in finding a necrotic tissue, determining an ischemic portion, and finding an infarct portion of a blood vessel. In addition, exposure to radiation can be avoided because no radioisotope is used, and there is another advantage that neither a particle accelerator nor an installation for RI experiments is required. Furthermore, image information obtained by $^{19}F$-MRI is superior to that obtained by the method of nuclear medicine, in respect to contrast and resolution in many cases.

The $^{19}F$-MRI contrast medium of the present invention can also be applied to tissue specific imaging and imaging of a functionally abnormal portion, such as a metabolic abnormality, as described above.

Moreover, the polyamine metal complex used as the imaging active substance in the $^{19}F$-MRI contrast medium of the present invention can be used in the following manner, as well as administering it into a living body as a contrast medium. That is, by coating or mixing the polyamine metal complex on or into a catheter or an artificial organ, position information of the catheter or the artificial organ can be obtained by using MRI.

A novel polyamine metal complex for use in the above $^{19}F$-MRI contrast medium will be described below.

The polyamine complex compound provided by the present invention is a metal complex compound represented by the following formula and its physiologically acceptable salts :

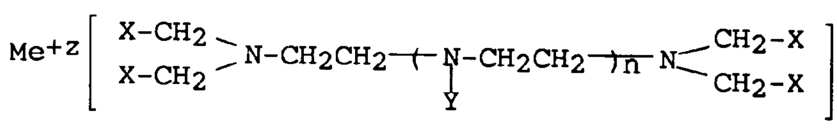

wherein Me, z, X, n, and Y have the following meanings;

Me : paramagnetic metal ion

z : the ionic valence of Me, a positive integer (preferably 2 or 3)

X : which may be the same or different and represents $-COO^-$, $-COOZ$, $-PO_3HZ$, $-CONHW$, or $-OH$ wherein Z and W are;

Z : a hydrogen atom or an organic or inorganic base equivalent or metal ion equivalent

W : a straight chain or branched chain alkyl group substituted with not less than one -OH group

n : an integer from 1 to 3

Y : when n = 1, Y is R wherein R is a substituent which has not less than one fluorine atom and may

contain X defined above, and when n = 2 or 3, at least one Y is R defined above and each of remaining Y's is $-CH_2X$, a lower alkyl group, or a hydrogen atom.

and wherein a ligand portion of the metal complex compound is coordinate-bonded to the paramagnetic metal ion so as to occupy coordination sites of the ion via at least some of nitrogen atoms and/or oxygen atoms which are contained in the ligand and can be coordinated.

The paramagnetic metal ion $Me^{+Z}$ in the polyamine metal complex of the present invention has already been described. Nitrogen atoms in the polyamine ligand and oxygen atoms of the hydroxyl group can contribute to a coordinating bond between the polyamine ligand and the paramagnetic metal. This hydroxyl group may be either a hydroxyl group contained in X (including X contained in Y) or a hydroxyl group contained in W.

If Z represents an inorganic base or an organic base, the base is used to obtain a complex in the form of a stable neutral salt because toxicity can be reduced in some cases by making the complex into the form of the neutral salt. Examples of the inorganic base are sodium, potassium, lithium, and so on. Examples of the organic base are various amines, such as N-methylglucamine, N,N-dimethylglucamine, ethanolamine, diethanolamine, tromethanmine, 3-amino-1,2-propanediol, and morpholine, and basic amino acids, such as lysine and arginine.

The length of the alkyl chain and the number of hydroxyl groups of the straight chain or branched chain alkyl group W substituted by one or more -OH groups are not particularly limited. This alkyl group W, however, preferably has one to six carbon atoms and one to five hydroxyl groups. Preferable examples of W are a hydroxymethyl group, a 2-hydroxyethyl group, a 2,3-dihydroxypropyl group, a dihydroxyisopropyl group, and a 2,3,4-trihydroxybutyl group. Some -OH groups contained in these hydroxylalkyl groups W may be coordinate-boded to the paramagnetic metal ion Me. In addition, a sugar residual group or a hydrophilic group having a polyether structure can be used as W instead of the hydroxyalkyl group.

R, at least one of which is contained in Y, i.e., the substituent having one or more fluorine atoms is used to introduce $^{19}F$ as the detectable nucleus of $^{19}F$-MRI into molecules of the polyamine complex compound. R is therefore not particularly limited so far as it can achieve this purpose. R is, however, preferably a straight chain or branched chain alkyl group substituted by one or more fluorine atoms. R may be of course a perfluoroalkyl group in which all hydrogen atoms are substituted by fluorine. The number of carbon atoms of these alkyl groups is preferably one to ten. The number of fluorine atoms in a molecule is preferably 1 to 20. Examples of such a fluorine-substituted alkyl group are a trifluoromethyl group, a perfluoroethyl group, a 2,2,2-trifluoroethyl group, a perfluoropropyl group, a bis(trifluoromethyl)methyl group, a tris(trifluoromethyl)methyl group, a 2,2,3,3,3-pentafluoropropyl group, and a perfluorobutyl group. These fluorine-substituted alkyl groups may contain a functional group, such as a hydroxyl group, like a bis(trifluoromethyl)hydroxymethyl group. In addition to the above fluorine-substituted alkyl groups, an aryl group or an aralkyl group, in which one or more aromatic hydrogen atoms are substituted with fluorine atoms, can be preferably used as R. These alkyl, aryl, and aralkyl groups may contain X defined above.

The general formulas of the above preferable examples of R are as follows.

$-R_{1F}$;
$-R_2-Ar_F$, $-R_2-\Phi-R_2-Ar_F$;
$-SO_2-R_{1F}$, $-SO_2-R_2-R_{1F}$, $-SO_2-Ar_F$, $-SO_2-R_2-Ar_F$;
$-CO-R_{1F}$, $-CO-R_2-R_{1F}$, $-CO-Ar_F$, $-CO-R_2-Ar_F$;
$-R_2-NH-SO_2-R_{1F}$, $-R_2-NH-SO_2-Ar_F$, $-R_2-NH-SO_2-R_2-Ar_F$;
$-R_2-SO_2-NH-R_{1F}$, $-R_2-SO_2-NH-Ar_F$, $-R_2-SO_2-NH-R_2-Ar_F$;
$-R_2-NH-CO-R_{1F}$, $-R_2-NH-CO-Ar_F$, $-R_2-NH-CO-R_2-Ar_F$;
$-R_2-CO-NH-R_{1F}$, $-R_2-CO-NH-Ar_F$, $-R_2-CO-NH-R_2-Ar_F$;
$-R_2-S-R_{1F}$, $-R_2-S-Ar_F$, $-R_2-S-R_2-Ar_F$;
$-R_2-O-R_{1F}$, $-R_2-O-Ar_F$, $-R_2-O-R_2-Ar_F$;
$-R_2-NH-R_{1F}$, $-R_2-NH-R_2-Ar_F$;
$-R_2-N(R_3)-R_{1F}$, $-R_2-N(R_3)-R_2-Ar_F$;
$-R_2-N(X)-R_{1F}$, $-R_2-N(X)-R_2-Ar_F$;
$-R_2-N(R_{1F})-R_2-Ar_F$, and
$-R_2-N(R_2-Ar_F)_2$,

wherein X represents the same meaning as defined above, and $-R_{1F}$, $-R_2-$, $-R_3$, $-\Phi-$, and $-Ar_F$ have the following meanings;

$-R_{1F}$: a straight chain or branched chain alkyl group which is substituted with one or more fluorine atoms and may contain X
$-R_2-$: a saturated or unsaturated hydrocarbon chain
$-R_3$ : a lower alkyl group
$-\Phi-$ : a phenylene group

$-Ar_F$:

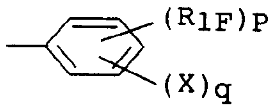

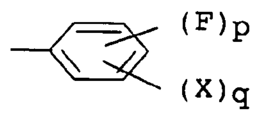

(p represents an integer from 1 to 5, and q represents 0 or an integer from 1 to 4).

The above polyamine metal complex has various excellent characteristic features already described above. First, $^{19}F$ and the paramagnetic metal ion exist in the same molecule. Second, the polyamine complex has a structure in which a plurality of fluorine atoms can be introduced easily into each molecule. Third, the polyamine complex has a good molecular symmetry by which the chemical shifts of a plurality of fluorine atoms become essentially the same. Fourth, polyamine is an extremely superior ligand, and its complex with a paramagnetic metal is very stable. Fifth, since it is easy to introduce substituents into the polyamine ligand, various chemical modifications can be performed easily for the polyamine ligand.

In addition to the above characteristic features, the sixth characteristic feature of the polyamine metal complex compound of the present invention is that the osmotic pressure of the entire molecule can be kept relatively low because the ligand can have a large number of hydroxyl groups at its terminal. Unlike an amino group or a carboxyl group, a hydroxyl group does not dissociate into ions in an aqueous solution. Therefore, the osmotic pressure can be kept low even if a hydroxyl group is used as a hydrophilic group. This characteristic feature makes it possible to avoid side-effects caused by a high osmotic pressure of the contrast medium when it is administered to a living body.

The polyamine metal complex compound of the present invention can be manufactured easily by using materials, such as commercially available polyamine and aminoalcohol, through reactions well known to those skilled in the art in accordance with synthesis routes A and B below.

<u>Synthesis route A:</u>

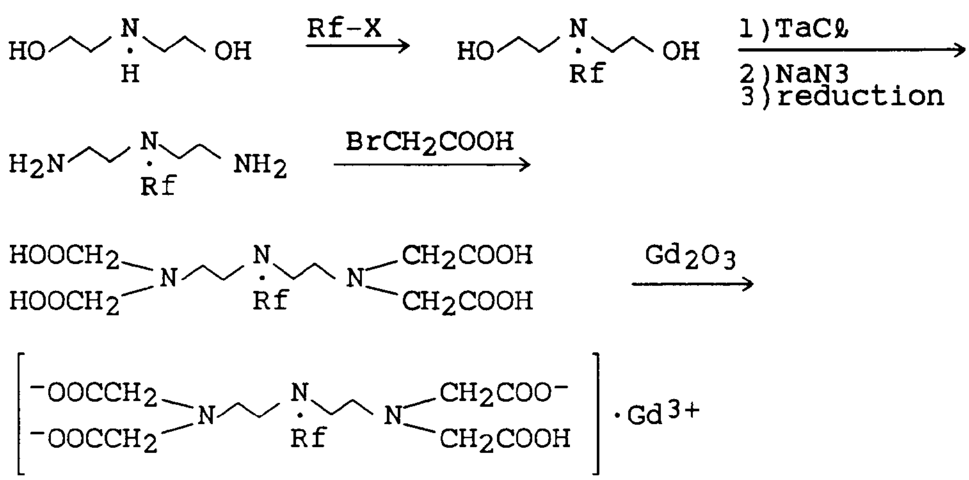


Rf: fluoro alkyl group

Synthesis route B:

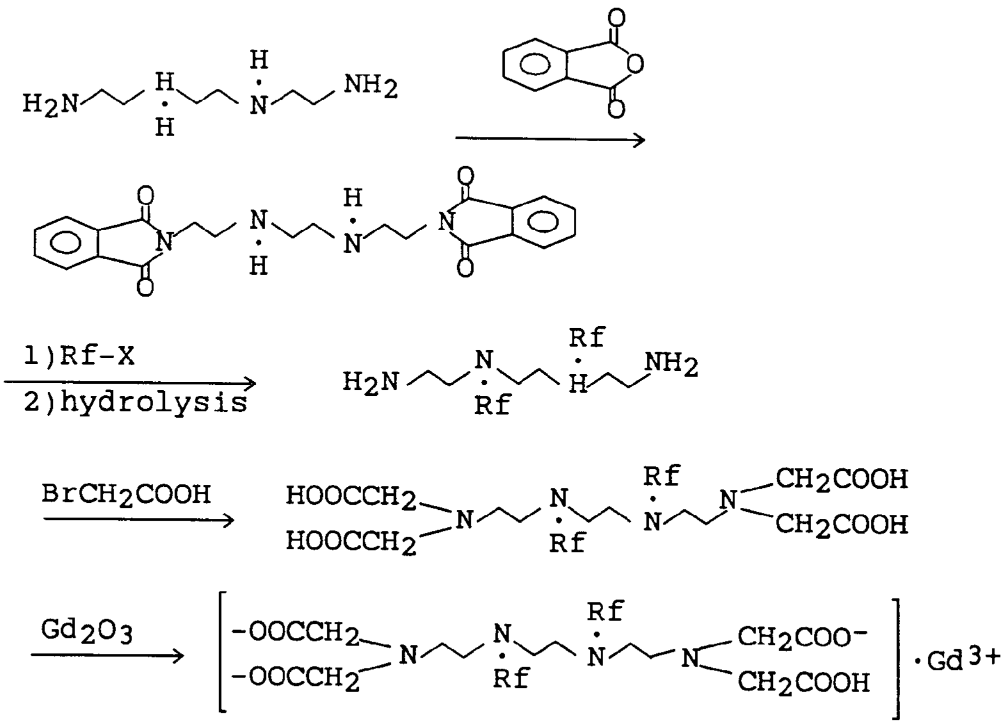


The final compound synthesized by each of the above routes can be isolated and purified by regular methods used in organic syntheses, such as an ion exchanging treatment, an activated carbon treatment, solvent precipitation, evaporation to dryness, recrystallization, and spray drying.

In order to obtain the final product in the form of various salts or in order to match the final product to the pH of a living body, an equivalent amount of counter ions may be added to a free compound to cause a reaction, and then the compound need only be isolated and purified in the same manner as described above.

The present invention will be described in more detail below by way of its examples, but these examples are presented merely for the purpose of illustrating the present invention, so it is to be understood that the present invention is not limited to these examples.

Example 1

Synthesis of gadolinium complex (compound-1) with N'-(2,2,2-trifluoroethyl)diethylenetriamine-N,N,N",N"-tetraacetic acid

The chemical structure of the compound-1 was as follows:

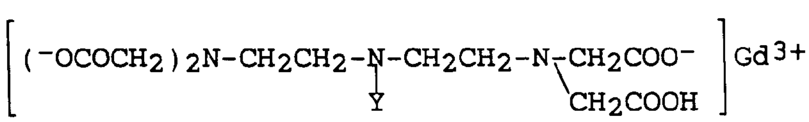

wherein Y = $-CH_2-CF_3$.

<Synthesis of ligand>

[Reaction 1]: 100 g (951 mmols) of diethanolamine were dissolved in 400 mℓ of dry tetrahydrofuran, and 92 g (951 mmols) of triethylamine were added to the solution. Thereafter, 170 g (793 mmols) of trifluoromethylsulfonyloxy-2,2,2-trifluoroethane were added dropwise into the resultant solution under cooling. After being reacted for three hours, the reaction mixture was poured into distilled water, and the resultant solution was extracted with ethyl acetate. The organic layer was washed with saturated brine, dried over anhydrous sodium sulfate, and then dried under reduced pressure. The yield was 87%.

[Reaction 2]: 120 g (690 mmols) of the product of Reaction 1 were dissolved in 600 mℓ of pyridine, and

317 g (1,664 mmols) of p-toluenesulfonyl chloride were added to the solution under cooling in an ice bath over one hour. The resultant solution was reacted at 5°C for two hours. The reaction mixture was poured in 1 ℓ of 1 N hydrochloric acid containing broken ice while stirring strongly. The precipitated solid was filtered out under reduced pressure, washed with water, and dried in a vacuum desiccator. The yield was 80%.

[Reaction 3]: 274 g (552 mmols) of the product of Reaction 2 were dissolved in 600 mℓ of ethanol, and the solution was mixed into 500 mℓ of distilled water in which 62 g (947 mmols) of sodium azide had been dissolved in advance. The resultant solution mixture was refluxed under heating for two hours. The reaction mixture was evaporated to dryness under reduced pressure and added with 200 mℓ of distilled water. The resultant solution was extracted with 300 mℓ of ethyl acetate. The organic layer was washed with 150 mℓ of distilled water and 150 mℓ of saturated brine, dried over anhydrous sodium sulfate, and concentrated under reduced pressure. The yield was 90%.

[Reaction 4]: 118 g (497 mmols) of the product of Reaction 3 were dissolved in 200 mℓ, of methanol, and 10 g of 10% palladium carbon were added to the solution. The resultant solution was subjected to catalytic hydrogen reduction at normal pressure for five hours. The catalyst was filtered off, and the resultant filtrate was concentrated under reduced pressure. The yield was 83%.

[Reaction 5]: 76 g (412 mmols) of the product of Reaction 4 were dissolved in 200 mℓ of dimethylformamide, and 287 mℓ (2,060 mmols) of triethylamine were added to the solution. Subsequently, 344 g (2,060 mmols) of ethyl bromoacetate were added dropwise into the resultant solution over 30 minutes. After being reacted at room temperature for two hours, the resultant solution was concentrated under reduced pressure and purified through silica gel column chromatography using a solvent mixture of ethyl acetate/n-hexane = 3/7 as an eluting solution. The yield was 75%.

[Reaction 6]: 163 g (309 mmols) of the product of Reaction 5 were dissolved in 150 mℓ of ethanol, and 1,236 mℓ of 1 N sodium hydroxide were added to the solution. The resultant solution was reacted under stirring at room temperature for 15 hours. The reaction mixture was concentrated under reduced pressure and treated by using a cation exchange resin (Amberlite IR-120B), thereby preparing a ligand compound. The yield was 85%.

The physico-chemical data of the obtained ligand compound was as follows. Note that the spectral data was measured under the following conditions. Nuclear magnetic resonance analysis (NMR)

Apparatus : EX-90 (90 MHz) manufactured by Nihon Densi K.K.
Measurement solvent : heavy water
Standard substances : DSS ($^1H$)
trifluoroacetic acid ($^{19}F$)

Infrared spectroscopic analysis (IR)
Apparatus : IR-810 manufactured by Nihon Bunko K.K.
Measurement method : KBr tablet method

Mass spectrometric analysis (MS)
Apparatus : D-300 manufactured by Nihon Densi K.K.
Ionizing method : FAB (glycerin matrix containing hydrochloric acid)

. Molecular formula: $C_{14}H_{22}N_3O_8F_3$
. $^1H$-NMR (δppm, $D_2O$):
2.8 (m, $CH_2N$), 3.2 (m, $CH_2CF_3$), 3.5 (s, $CH_2CO$)
. $^{19}F$-NMR (δppm, $D_2O$): -68.1
. IR ($\nu cm^{-1}$): 2600-3400, 1720-1760 (COOH), 1240 ($CF_3$)
. FAB-MS (M/Z): 418 ($MH^+$)

<Synthesis of complex>

[Reaction 7]: 10 g (24 mmols) of the product of Reaction 6 were dissolved in 100 mℓ of distilled water containing 48 mℓ of 1 N sodium hydroxide, and 8.7 g (24 mmols) of gadolinium oxide were added to the solution. The resultant solution was reacted under heating at 80°C for three hours. The reaction mixture was filtered to remove unreacted gadolinium oxide, and the filtrate was evaporated to dryness under reduced pressure. The resultant material was treated by using a cation exchange resin (Amberlite IR-120B) and dried under reduced pressure. The yield was 98%.

Note that the yield of the complexation reaction was obtained from the weight and the metal quantity determined by ICP (Inductively Coupled Plasma emission spectroscopy). 14.5 g of the end product were obtained. The yield of the overall process was 32%.

Example 2

Synthesis of gadolinium complex (compound-2) with N'-[bis(trifluoromethyl)methyl]diethylenetriamine-N,N,N",N"-tetraacetic acid

Chemical structure of the compound-2:

The same as the compound-1;

except that Y = $-CH-(CF_3)_2$

<Synthesis of ligand>

Reactions 1 to 6 were performed following the same procedures as in Example 1 except that 238 g (793 mmols) of trifluoromethylsulfonyloxy[bis(trifluoromethyl)]methane were used in place of 170 g (793 mmols) of trifluoromethylsulfonyloxy-2,2,2-trifluoroethane in Reaction 1, thereby obtaining a ligand compound. The yields in Reactions 1 to 6 were as follows.

[Reaction 1] = 86%; [Reaction 2] = 82%;

[Reaction 3] = 89%; [Reaction 4] = 80%;

[Reaction 5] = 73%; [Reaction 6] = 83%

The physico-chemical data of the obtained ligand compound was as follows.

. Molecular formula: $C_{15}H_{21}N_3O_8F_6$

. $^1H$-NMR (δppm, $D_2O$):

2.7 (m, CH), 3.0 (m, $CH_2N$), 3.5 (s, $CH_2CO$)

. $^{19}F$-NMR (δppm, $D_2O$): -70.4

. IR (νcm$^{-1}$): 2600-3400, 1720-1760 (COOH), 1260 ($CF_3$)

. FAB-MS (M/Z): 486 ($MH^+$)

<Synthesis of complex>

The reaction was performed following the same procedures as for Reaction 7 of Example 1 by using the ligand compound obtained as described above. The yield was 96%. 13.6 g of the end product were obtained. The yield of the overall process was 29%.

Example 3

Synthesis of gadolinium complex (compound-3) with N'-[4,4,4-tris(trifluoromethyl)butyl] diethylenetriamine-N,N,N",N"-tetraacetic acid

Chemical structure of the compound-3:

The same as the compound-1;

except that Y = $-CH_2CH_2CH_2C-(CF_3)_3$

<Synthesis of ligand>

Reactions 1 to 6 were performed following the same procedures as in Example 1 except that 212 g (793 mmols) of trifluoromethylsulfonyloxy-4,4,4-tris(trifluoromethyl)butane were used in place of 170 g (793 mmols) of trifluoromethylsulfonyloxy-2,2,2-trifluoroethane in Reaction 1, thereby obtaining a ligand compound. The yields in Reactions 1 to 6 were as follows.

[Reaction 1] = 85%; [Reaction 2] = 79%;

[Reaction 3] = 87%; [Reaction 4] = 90%;

[Reaction 5] = 80%; [Reaction 6] = 83%

The physico-chemical data of the obtained ligand compound was as follows.

. Molecular formula: $C_{16}H_{26}N_3O_8F_3$

. $^1H$-NMR (δppm, $D_2O$):

2.4-2.7 (m, $CH_2CH$ $CCF_3$), 2.7 (m, $CH_2N$), 3.7 (s, $CH_2CO$)

. $^{19}F$-NMR (δppm, $D_2O$): -69.3

. IR (νcm$^{-1}$): 2600-3400, 1720-1760 (COOH), 1210 ($CF_3$)

. FAB-MS (M/Z): 446 ($MH^+$)

<Synthesis of complex>

The reaction was performed following the same procedures as for Reaction 7 of Example 1 by using the ligand compound obtained as described above. The yield was 94%. 13.6 g of the end product were obtained. The yield of the overall process was 33%.

Example 4

Synthesis of gadolinium complex (compound-4) with N'-(1-carboxy-3,3,3-trifluoropropyl) diethylenetriamine-N,N,N",N"-tetraacetic acid

Chemical structure of the compound-4:

The same as the compound-1;

except that Y = $-CH(COOH)CH_2-CF_3$

<Synthesis of ligand>

[Reaction 1]: 100 g (951 mmols) of diethanolamine and 65.7 g (457 mmols) of potassium carbonate were dissolved in 200 mℓ of distilled water. 213 g (795 mmols) of 2-iodo-4,4,4-trifluoroacetic acid dissolved in 300 mℓ of tetrahydrofuran were added dropwise into the resultant solution under cooling over 30 minutes. After the resultant solution was reacted at room temperature for five hours, the organic layer was removed, and the remaining aqueous layer was extracted twice with 200 mℓ of ether. The extract and the organic layer were taken together and washed with saturated brine, dried over anhydrous sodium sulfate, and concentrated under reduced pressure. The yield was 90%.

A ligand compound was prepared following the same procedures as for Reactions 2 to 6 in Example 1 except that the above reaction product was used. The yields in the individual reactions were as follows.

[Reaction 2] = 83%; [Reaction 3] = 92%;

[Reaction 4] = 93%; [Reaction 5] = 82%;

[Reaction 6] = 82%

The physico-chemical data of the ligand compound obtained by Reaction 6 was as follows.

. Molecular formula: $C_{16}H_{24}N_3O_{10}F_3$

. $^1$H-NMR (δppm, $D_2O$):

1.9 (m, $CCH_2CF_3$), 3.0 (m, $CH_2N$), 3.3 (s, $CH_2CO$), 3.6 (m, CH)

. $^{19}$F-NMR (δppm, $D_2O$): -65.4

. IR (νcm$^{-1}$): 2600-3400, 1720-1760 (COOH), 1260 ($CF_3$)

. FAB-MS (M/Z): 476 ($MH^+$)

<Synthesis of complex>

[Reaction 7]: The reaction was performed following the same procedures as for Reaction 7 of Example 1 except that the product of Reaction 6 was used as a starting material. The yield was 98%. 13.9 g of the end product were obtained. The yield of the overall process was 42%.

Example 5

Synthesis of gadolinium complex (compound-5) with N',N"-bis(2,2,2-trifluoroethyl) triethylenetetramine-N,N,N"',N"'-tetraacetic acid

The chemical structure of the compound-5 was as follows:

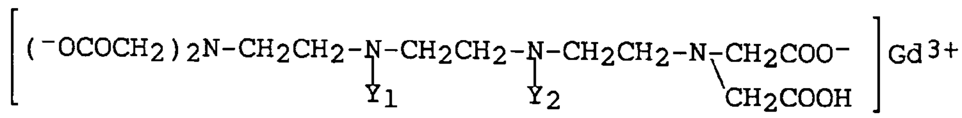

wherein $Y_1$ and $Y_2$ = $-CH_2-CF_3$.

<Synthesis of ligand>

[Reaction 1]: 500 g (3.42 mols) of triethylenetetramine and 1,064 g (7.18 mols) of phthalic anhydride were

dissolved in 20 ℓ of chloroform, and the resultant solution was refluxed under heating for 20 hours. After the reaction mixture was cooled, the precipitated solid was filtered out, washed with chloroform, and dried. The yield was 97%.

[Reaction 2]: 100 g (275 mmols) of the product of Reaction 1 were dissolved in 300 mℓ of dry tetrahydrofuran, and 28 g (275 mmols) of triethylamine were added to the solution. Thereafter, 140 g (605 mmols) of trifluoromethylsulfonyloxy-2,2,2-trifluoroethane were added dropwise into the resultant solution under cooling. After being reacted for three hours, the solution was poured into distilled water, and the resultant solution was extracted with ethyl acetate. The organic layer was washed with saturated brine, dried over anhydrous sodium sulfate, and then dried under reduced pressure. The yield was 86%.

[Reaction 3]: 135 g (237 mmols) of the product of Reaction 2 were suspended in ethanol containing hydrazine hydrate, and the suspension was refluxed under heating for four hours. The reaction mixture was concentrated under reduced pressure and subjected to a cation exchange column (Amberlite IR-120B), thereby obtaining the object product from a fraction eluted by ammonia water. The yield was 85%.

[Reaction 4]: The reaction was performed following the same procedures as for Reaction 5 of Example 1 except that the product of Reaction 3 was used as a starting material. The yield was 80%.

[Reaction 5]: The reaction was performed following the same procedures as for Reaction 6 of Example 1 except that the product of Reaction 4 was used as a starting material, thereby obtaining a ligand compound. The yield was 70%.

The physico-chemical data of the ligand compound obtained by Reaction 5 was as follows.

. Molecular formula: $C_{18}H_{28}N_4O_8F_6$

. $^1H$-NMR (δppm, $D_2O$):

2.8 (m, $CH_2N$), 3.1 (m, $CH_2CF_3$), 3.5 (s, $CH_2CO$)

. $^{19}F$-NMR (δppm, $D_2O$): -74.1

. IR ($\nu cm^{-1}$): 2600-3400, 1720-1760 (COOH), 1290 ($CF_3$)

. FAB-MS (M/Z): 408 ($MH^+$)

<Synthesis of complex>

[Reaction 6]: The reaction was performed following the same procedures as for Reaction 7 of Example 1 except that the product of Reaction 5 was used as a starting material. The yield was 97%. 14.9 g of the end product were obtained. The yield of the overall process was 39%.

Example 6

Synthesis of gadolinium complex (compounds-6) with N'-(2,2,2-trifluoroethyl) triethylenetetramine-N,N,N",N"',N"'-pentaacetic acid

Chemical structure of the compound-6:

The same as the compound-5;

except that $Y_1$ = $-CH_2-CF_3$, $Y_2$ = $-CH_2COOH$

<Synthesis of ligand>

[Reaction 1]: 100 g (275 mmols) of the product of Reaction 1 in Example 5 were dissolved in 300 mℓ of dry tetrahydrofuran, and 28 g (275 mmols) of triethylamine were added to the solution. Thereafter, 70 g (303 mmols) of trifluoromethylsulfonyloxy-2,2,2-trifluoroethane were added dropwise into the resultant solution. After being reacted for three hours, the solution was poured into distilled water, and the resultant solution was extracted with ethyl acetate. The organic layer was washed with saturated brine, dried over anhydrous sodium sulfate, and then evaporated to dryness under reduced pressure. The yield was 72%.

[Reaction 2]: The reaction was performed following the same procedures as for Reaction 3 of Example 5 except that the product of Reaction 1 was used as a starting material. The yield was 79%.

[Reaction 3]: The reaction was performed following the same procedures as for Reaction 5 of Example 1 except that the product of Reaction 2 was used as a starting material. The yield was 85%.

[Reaction 4]: The reaction was performed following the same procedures as for Reaction 6 of Example 1 except that the product of Reaction 3 was used as a starting material, thereby obtaining a ligand compound. The yield was 81%.

The physico-chemical data of the obtained ligand compound was as follows.

. Molecular formula: $C_{18}H_{29}N_4O_{10}F_3$

. $^1H$-NMR (δppm, $D_2O$):

2.9 (m, $CH_2N$), 3.2 (m, $CH_2CF_3$), 3.7 (s, $CH_2CO$)
. $^{19}$F-NMR (δppm, $D_2O$): -73.6
. IR (νcm$^{-1}$): 2600-3400, 1720-1760 (COOH), 1240 ($CF_3$)
. FAB-MS (M/Z): 384 ($MH^+$)

<Synthesis of complex>

[Reaction 5]: The reaction was performed following the same procedures as for Reaction 7 of Example 1 except that the product of Reaction 4 was used as a starting material. The yield was 98%. 15.2 g of the end product were obtained. The yield of the overall process was 37%.

Example 7

Synthesis of gadolinium complex (compound-7) with N'-[4-(trifluoromethyl)benzyl]diethylenetriamine-N,N,N",N"-tetraacetic acid

The chemical structure of the compound-7 was as follows:

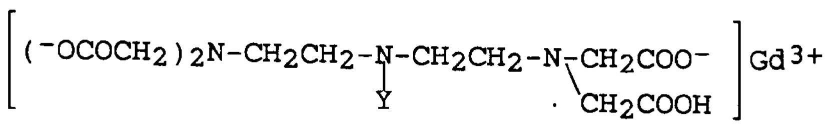

wherein

Y = 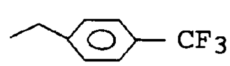

<Synthesis of ligand>

[Reaction 1]: 100 g (951 mmols) of diethanolamine and 65.7 g (475 mmols) of potassium carbonate were dissolved in 200 mℓ of distilled water. 190 g (795 mmols) of 4-(trifluoromethyl)benzyl bromide dissolved in 300 mℓ of tetrahydrofuran were added dropwise into the resultant solution under cooling over 30 minutes. After the resultant solution was reacted at room temperature for five hours, the organic layer was removed, and the remaining water layer was extracted twice with 200 mℓ of ether. The extract and the organic layer were taken together and washed with saturated brine, dried over anhydrous sodium sulfate, and concentrated under reduced pressure. The yield was 91%.

A ligand compound was prepared following the same procedures as for Reactions 2 to 6 of Example 1 except that the above reaction product was used. The yields in the individual reactions were as follows.

[Reaction 2] = 81%; [Reaction 3] = 87%;
[Reaction 4] = 82%; [Reaction 5] = 77%;
[Reaction 6] = 84%

The physico-chemical data of the ligand compound obtained by Reaction 6 was as follows.

. Molecular formula: $C_{20}H_{26}N_3O_8F_3$
. $^1$H-NMR (δppm, $D_2O$):
2.7 (m, $CH_2N$), 3.2 (s, $CH_2CO$), 3.7 (m, ϕ-$CH_2$), 7.4-7.8 (m, ϕ)
. $^{19}$F-NMR (δppm, $D_2O$): -65.1
. IR (νcm$^{-1}$): 2600-3400, 1720-1760 (COOH), 1240 ($CF_3$)
. FAB-MS (M/Z): 494 ($MH^+$)

<Synthesis of complex>

[Reaction 7]: The reaction was performed following the same procedures as for Reaction 7 of Example 1 except that the product of Reaction 6 was used as a starting material. The yield was 96%. 14.1 g of the end product were obtained. The yield of the overall process was 33%.

Example 8

Synthesis of gadolinium complex (compound-8) with N'-[3,5-bis(trifluoromethyl)benzyl] diethylenetriamine-N,N,N",N"-tetraacetic acid

Chemical structure of the compound-8:

The same as the compound-7;

except that

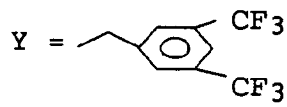

<Synthesis of ligand>

Reactions 1 to 6 were performed following the same procedures as in Example 7 except that 244 g (795 mmols) of 3,5-bis(trifluoromethyl)benzyl bromide were used in place of 190 g (795 mmols) of 4-(trifluoromethyl)benzyl bromide in Reaction 1, thereby obtaining a ligand compound. The yields in Reactions 1 to 6 were as follows.

[Reaction 1] = 87%; [Reaction 2] = 83%;
[Reaction 3] = 91%; [Reaction 4] = 94%;
[Reaction 5] = 79%; [Reaction 6] = 86%

The physio-chemical data of the ligand compound obtained by Reaction 6 was as follows.

. Molecular formula: $C_{21}H_{25}N_3O_8F_6$
. $^1$H-NMR (δppm, $D_2O$):
2.6 (m, $CH_2N$), 3.6 (s, $CH_2CO$), 3.7 (m, ϕ-$CH_2$), 7.9-8.2 (m, ϕ)
. $^{19}$F-NMR (δppm, $D_2O$): -65.2
. IR (ν$cm^{-1}$): 2600-3400, 1720-1760 (COOH), 1250 ($CF_3$)
. FAB-MS (M/Z): 562 ($MH^+$)

<Synthesis of complex>

The reaction was performed following the same procedures as for Reaction 7 of Example 1 by using the ligand compound obtained as described above. The yield was 94%. 13.6 g of the end product were obtained. The yield of the overall process was 39%.

Example 9

Synthesis of gadolinium complex (compound-9) with N'-[4-[tris(trifluoromethyl)methyl]benzyl] diethylenetriamine-N,N,N",N"-tetraacetic acid

Chemical structure of the compound-9:

The same as the compound-7;

except that

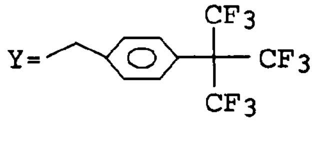

<Synthesis of ligand>

Reactions 1 to 6 were performed following the same procedures as in Example 7 except that 309 g (795 mmols) of 4-[tris(trifluoromethyl)methyl]benzyl bromide were used in place of 190 g (795 mmols) of 4-(trifluoromethyl)benzyl bromide in Reaction 1, thereby obtaining a ligand compound. The yields in Reactions 1 to 6 were as follows.

[Reaction 1] = 81%; [Reaction 2] = 87%;
[Reaction 3] = 93%; [Reaction 4] = 92%;

[Reaction 5] = 80%; [Reaction 6] = 88%

The physico-chemical data of the ligand compound obtained as described above was as follows.

. Molecular formula: $C_{23}H_{26}N_3O_8F_9$

. $^1$H-NMR (δppm, $D_2O$):

2.8 (m, $CH_2N$ x4), 3.3 (s, $CH_2CO$), 3.7 (m, ϕ-$CH_2$), 7.2-7.3 (m, ϕ)

. $^{19}$F-NMR (δppm, $D_2O$): -63.8

. IR (νcm$^{-1}$): 2600-3400, 1720-1760 (COOH), 1240 ($CF_3$)

. FAB-MS (M/Z): 644 ($MH^+$)

<Synthesis of complex>

The reaction was performed following the same procedures as for Reaction 7 of Example 1 by using the above ligand compound. The yield was 95%. 13.1 g of the end product were obtained. The yield of the overall process was 40%.

Example 10

Synthesis of gadolinium complex (compound-10) with N'-(2,3,4,5,6-pentafluorobenzyl) diethylenetriamine-N,N,N",N"-tetraacetic acid

Chemical structure of the compound-10:

The same as the compound-7;

except that

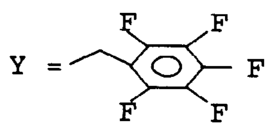

<Synthesis of ligand>

Reactions 1 to 6 were performed following the same procedures as in Example 7 except that 207 g (795 mmols) of 2,3,4,5,6-pentafluorobenzyl bromide were used in place of 190 g (795 mmols) of 4-(trifluoromethyl)benzyl bromide in Reaction 1, thereby obtaining a ligand compound. The yields in Reactions 1 to 6 were as follows.

[Reaction 1] = 92%; [Reaction 2] = 82%;

[Reaction 3] = 90%; [Reaction 4] = 89%;

[Reaction 5] = 83%; [Reaction 6] = 89%

The physico-chemical data of the ligand compound obtained as described above was as follows.

. Molecular formula: $C_{19}H_{22}N_3O_8F_5$

. $^1$H-NMR (δppm, $D_2O$):

2.7 (m, $CH_2N$), 3.1 (s, $CH_2CO$), 3.6 (m, ϕ-$CH_2$)

. 19F-NMR (δppm, D2O): -160 to -150

. IR (νcm$^{-1}$): 2600-3400, 1720-1760 (COOH)

. FAB-MS (M/Z): 516 ($MH^+$)

<Synthesis of complex>

The reaction was performed following the same procedures as for Reaction 7 of Example 1 by using the above ligand compound. The yield was 92%. 13.9 g of the end product were obtained. The yield of the overall process was 41%.

Example 11

Synthesis of gadolinium complex (compound-11) of N'-[3,5-bis(trifluoromethyl)benzyl] diethylenetriamine-N,N,N",N"-tetrakis(acetic-2,3-dihydroxypropylamide)

The chemical structure of the compound-11 was as follows:

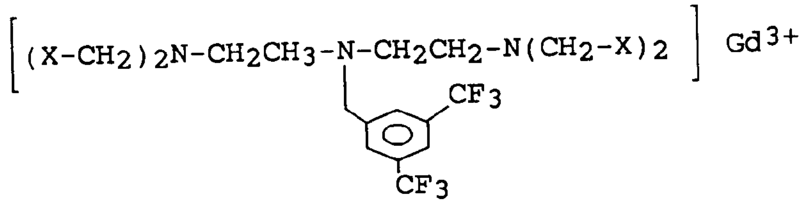

wherein

X = 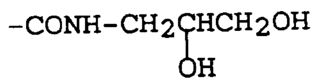

<Synthesis of ligand>

50 g (59 mmols) of the product of Reaction 5 in Example 8 were mixed in 27 g (293 mmols) of 2,3-dihydroxypropylamine, and the resultant mixture was reacted under stirring at 100°C for two hours. The reaction product was dissolved in a small amount of water, and the resultant solution was treated with a cation exchange resin (Amberlite IR-120B). The yield was 79%.

The physico-chemical data of the ligand compound obtained by Reaction 1 was as follows.

. Molecular formula: $C_{33}H_{53}N_7O_{12}F_6$

. $^1$H-NMR (δppm, $D_2O$):

2.9 (m, $CH_2N$), 3.6 (m, ϕ-$CH_2$), 3.7 (s, $CH_2CO$), 3.6-4.2 (m, amide side chain), 7.6-7.9 (m, ϕ)

. $^{19}$F-NMR (δppm, $D_2O$): -64.8

. IR (νcm$^{-1}$): 3200-3600 (OH), 1650 (CONH), 1290 ($CF_3$)

. FAB-MS (M/Z): 854 ($MH^+$)

<Synthesis of complex>

The reaction was performed following the same procedures as for Reaction 7 of Example 1 by using the ligand compound obtained as described above as a starting material. The yield was 90%. 12.4 g of the end product were obtained. The yield of the overall process was 30%.

Example 12

Synthesis of gadolinium complex (compound-12) of N'-[3,5-bis(trifluoromethyl)benzyl] diethylenetriamine-N,N,N",N"-tetrakis(acetic 2,3,4-trihydroxybutylamide)

Chemical structure of the compound-12:

The same as the compound-11;

except

x = 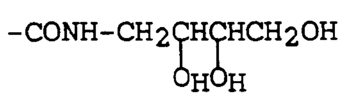

<Synthesis of ligand>

50 g (51 mmols) of the product of Reaction 6 in Example 8 were mixed with 31 g (257 mmols) of 2,3,4-trihydroxybutylamine, and the resultant mixture was reacted under stirring at 100°C for two hours. The reaction product was dissolved in a small amount of water, and treated with a cation exchange resin (Amberlite IR-120B). The yield was 80%.

The physico-chemical data of the ligand compound obtained by Reaction 1 was as follows.

. Molecular formula: $C_{37}H_{61}N_7O_{16}F_6$

. $^1$H-NMR (δppm, $D_2O$):

2.6 (m, $CH_2N$), 3.5 (s, $CH_2CO$), 3.5-4.3 (m, amide side chain), 3.8 (m, ϕ-$CH_2$), 7.9-8.1 (m, ϕ)

. $^{19}$F-NMR (δppm, $D_2O$): -65.2

. IR (νcm$^{-1}$): 3200-3600 (OH), 1650 (COOH), 1270 ($CF_3$)

. FAB-MS (M/Z): 974 ($MH^+$)

<Synthesis of complex>

The reaction was performed following the same procedures as for Reaction 7 of Example 1 by using the ligand compound obtained as described above as a starting material. The yield was 91%. 12.1 g of the end product were obtained. The yield of the overall process was 31%.

Example 13

Synthesis of gadolinium complex (compound-13) with N'-[3,5-bis(trifluoromethyl) benzyl]diethylenetriamine-N,N,N",N"-tetrakis(acetic-1,3-dihydroxyisopropylamide)
Chemical structure of the compound-13:
The same as the compound-11;
except that X = $-CONH-CH(CH_2OH)_2$

<Synthesis of ligand>

50 g (59 mmols) of the product of Reaction 5 in Example 8 were mixed with 27 g (293 mmols) of 1,3-dihydroxyisopropylamine, and the resultant mixture was reacted under stirring at 100°C for two hours. The reaction product was dissolved in a small amount of water, and treated with a cation exchange resin (Amberlite IR-120B). The yield was 73%.

The physico-chemical data of the ligand compound obtained by the reaction mentioned above was as follows.

. Molecular formula: $C_{35}H_{57}N_7O_{12}F_6$
. $^1$H-NMR (δppm, $D_2O$):
2.9 (m, $CH_2N$), 3.5 (s, $CH_2CO$), 3.8-4.0 (m, amide side chain), 3.8 (m, ϕ-$CH_2$), 8.1-8.3 (m, ϕ)
. $^{19}$F-NMR (δppm, $D_2O$): -70.6
. IR (ν$cm^{-1}$): 3200-3600 (OH), 1650 (CONH), 1280 ($CF_3$)
. FAB-MS (M/Z): 854 ($MH^+$)

<Synthesis of complex>

The complex synthesis reaction was performed following the same procedures as for Reaction 7 of Example 1 by using the ligand compound obtained as described above as a starting material. The yield was 93%. 12.0 g of the end product were obtained. The yield of the overall process was 28%.

Example 14

Synthesis of gadolinium complex (compound-14) with N'-[3,5-bis(trifluoromethyl)benzyl]-N, N,N",N"-tetra(hydroxymethyl)diethylenetriamine
Chemical structure of the compound-14:
The same as the compound-11;
except that X = -OH

<Synthesis of ligand>

50 g (110 mmols) of the product of Reaction 5 in Example 8 were dissolved in 150 mℓ of dry tetrahydrofuran, and 8 g (220 mmols) of lithium aluminum hydride suspended in dry tetrahydrofuran were added dropwise into the resultant solution. After being reacted under stirring at room temperature for one hour, the resultant solution was poured into ice water. The insolubles were filtered off, and the remaining aqueous solution was concentrated and dried. The resultant material was then purified through silica gel column chromatography using a solvent mixture of ethanol/chloroform = 4/6 as an eluting solution. The yield was 83%.

The physico-chemical data of the ligand compound obtained by the above reaction was as follows.

. Molecular formula: $C_{17}H_{25}N_3O_4F_6$
. $^1$H-NMR (δppm, $D_2O$):
2.7 (m, $CH_2N$), 4.2 (s, $CH_2OH$), 3.9 (m, ϕ-$CH_2$), 7.9-8.1 (m, ϕ)
. $^{19}$F-NMR (δppm, $D_2O$): -64.1

. IR ($\nu cm^{-1}$): 3200-3600 (OH), 1210 ($CF_3$)
. FAB-MS (M/Z): 450 ($MH^+$)

<Synthesis of complex>

The reaction was performed following the same procedures as for Reaction 7 of Example 1 by using the ligand compound obtained as described above as a starting material. The yield was 97%. 14.5 g of the end product were obtained. The yield of the overall process was 34%.

Example 15

Synthesis of gadolinium complex (compound-15) with N',N"-bis[3,5-bis(trifluoromethyl)benzyl] triethylenetetramine-N,N,N''',N'''-tetraacetic acid

The chemical structure of the compound-15 was as follows.

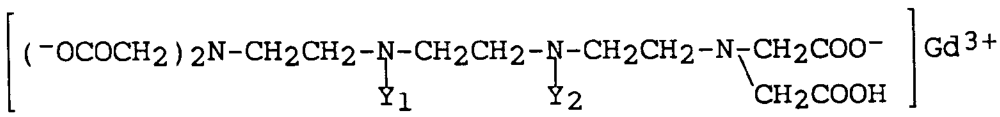

wherein

Y1 and Y2 = 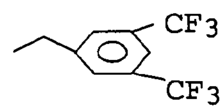

<Synthesis of ligand>

[Reaction 1]: 100 g (275 mmols) of the product of Reaction 1 in Example 5 were dissolved in 300 mℓ of dry tetrahydrofuran, and 28 g (275 mmols) of triethylamine were added to the solution. Thereafter, 228 g (605 mmols) of 3,5-bis(trifluoromethyl)benzyl bromide were added dropwise into the resultant solution under cooling. After being reacted for three hours, the solution was poured into distilled water, and the resultant solution was extracted with ethyl acetate. The organic layer was washed with saturated brine, dried over anhydrous sodium sulfate, and then evaporated to dryness under reduced pressure. The yield was 81%.

[Reaction 2]: The reaction was performed following the same procedures as for Reaction 3 of Example 5 except that the product of Reaction 1 was used as a starting material. The yield was 84%.

[Reaction 3]: The reaction was performed following the same procedures as for Reaction 5 of Example 1 except that the product of Reaction 2 was used as a starting material. The yield was 76%.

[Reaction 4]: The reaction was performed following the same procedures as for Reaction 6 of Example 1 except that the product of Reaction 3 was used as a starting material, thereby obtaining a ligand compound. The yield was 88%.

The physico-chemical data of the ligand compound obtained by Reaction 4 was as follows.

. Molecular formula: $C_{32}H_{34}N_4O_8F_{12}$
. $^1H$-NMR (δppm, $D_2O$):
  2.9 (m, $CH_2N$), 3.7 (s, $CH_2CO$), 4.0 (m, $\phi$-$CH_2$), 7.9-8.1 (m, $\phi$)
. $^{19}F$-NMR (κppm, $D_2O$): -66.2
. IR ($\nu cm^{-1}$): 2600-3400, 1720-1760 (COOH), 1240 ($CF_3$)
. FAB-MS (M/Z): 696 ($MH^+$)

<Synthesis of complex>

The reaction was performed following the same procedures as for Reaction 7 of Example 1 except that the product of Reaction 4 was used as a starting material. The yield was 94%. 12.1 g of the end product were obtained. The yield of the overall process was 41%.

Example 16

Synthesis of gadolinium complex (Compound-16) with N'-[3,5-bis(trifluoromethyl)benzyl] triethylenetetramine-N,N,N",N"',N"'-pentaacetic acid (compound-16)

Chemical structure of the compound-16:

The same as the compound-15;

except that

Y1 = 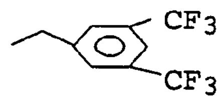 ,

Y2 = $-CH_2COOH$

<Synthesis of ligand>

A ligand compound was prepared following the same procedures as for Reactions 1 to 4 of Example 15 except that the addition amount of 3,5-bis(trifluoromethyl)benzyl bromide in Reaction 1 was changed to 114 g (303 mmols). The yields in the individual reactions were as follows.

[Reaction 1] = 76%; [Reaction 2] = 75%;

[Reaction 3] = 79%; [Reaction 4] = 85%;

The physico-chemical data of the ligand compound obtained by Reaction 4 was as follows.

. Molecular formula: $C_{25}H_{32}N_4O_{10}F_6$

. $^1$H-NMR (δppm, $D_2O$):

2.6 (m, $CH_2N$), 3.6 (s, $CH_2CO$), 3.7 (m, ϕ-CH2), 8.1-8.3 (m, ϕ)

. $^{19}$F-NMR (δppm, $D_2O$): -63.2

. IR (νcm$^{-1}$): 2600-3400, 1720-1760 (COOH), 1210 ($CF_3$)

. FAB-MS (M/Z): 528 ($MH^+$)

<Synthesis of complex>

The reaction was performed following the same procedures as for Reaction 7 of Example 1 except that the product of Reaction 4 was used as a starting material. The yield was 96%. 13.2 g of the end product were obtained. The yield of the overall process was 36%.

Example 17

Synthesis of gadolinium complex (compound-17) with N'-[4-[2-trans-(3,5-bis(trifluoromethyl)phenyl) vinyl]benzyl]diethylenetriamine-N,N,N",N"-tetraacetic acid

The chemical structure of the compound-17 was as follows:

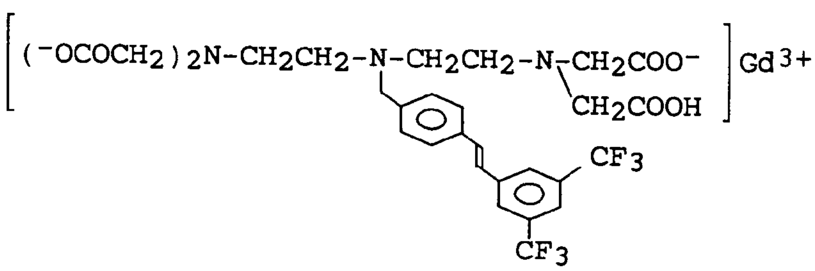

<Synthesis of ligand>

[Reaction 1]: 1 kg (2.7 mols) of 4-methylbenzyl bromide was dissolved in 3 ℓ of dry tetrahydrofuran, and 710 g of triphenylphosphine were added to the solution. The resultant solution was reacted at 80°C for one hour. After the solution was cooled, the precipitated solid was filtered out, washed with a small amount of tetrahydrofuran, and dried. The yield was 85%.

[Reaction 2]: 1,027 g (2.3 mols) of the product of Reaction 1 were dissolved in 3.5 ℓ of methanol, and 520 mℓ of a 28% methanol solution of sodium methoxide (2.7 mols) were added to the solution. 556 g (2.3 mols) of 3,5-bis(trifluoromethyl)benzaldehyde were added dropwise into the resultant solution under cooling down to ice temperature. After being reacted at room temperature for two hours, the resultant solution was concentrated under reduced pressure. The resultant residue was added with 1 ℓ of ethyl acetate, and washed twice with water and once with saturated brine, then dried over anhydrous sodium sulfate. The dried solution was purified through silica gel column chromatography using a solvent mixture of ethyl acetate/n-hexane = 1/19 as an eluting solution. The yield was 60%.

[Reaction 3]: 456 g (1.4 mols) of the product of Reaction 2 were dissolved in 3 ℓ of carbon tetrachloride, and 374 g (2.1 mols) of N-bromosuccinimide were added to the solution. The resultant solution was refluxed under heating for three hours. After the reaction mixture was filtered, the filtrate was concentrated under reduced pressure and purified through silica gel column chromatography using a solvent mixture of ethyl acetate/n-hexane = 1/19 as an eluting solution. The yield was 75%.

[Reaction 4]: 137 g (1.3 mols) of diethanolamine were dissolved in 400 mℓ of dry tetrahydrofuran, and 132 g (1.3 mols) of triethylamine were added to the solution. 430 g (1.1 mols) of the product of Reaction 3 were added dropwise into the resultant solution under cooling. After being reacted at room temperature for three hours, the solution was poured into distilled water, and the resultant solution was extracted with ethyl acetate. The organic layer was washed with saturated brine, dried over anhydrous sodium sulfate and then concentrated under reduced pressure. The resultant product was purified through silica gel column chromatography using a solvent mixture of ethyl acetate/n-hexane = 3/7 as an eluting solution. The yield was 80%.

Subsequently, Reactions 5 to 9 below were performed in this order to obtain a ligand compound.

[Reaction 5]: The same procedures as for Reaction 2 in Example 1. The yield was 82%.

[Reaction 6]: The same procedures as for Reaction 3 in Example 1. The yield was 89%.

[Reaction 7]: The same procedures as for Reaction 4 in Example 1. The yield was 85%.

[Reaction 8]: The same procedures as for Reaction 5 in Example 1. The yield was 75%.

[Reaction 9]: The same procedures as for Reaction 6 in Example 1. The yield was 80%.

The physico-chemical data of the ligand compound obtained as described above was as follows.

. Molecular formula: $C_{29}H_{31}N_3O_8F_6$

. $^1$H-NMR (δppm, $D_2O$):

2.8 (m, $CH_2N$), 3.5 (s, $CH_2CO$), 3.6 (m, $CH_2$), 6.8-7.3 (m, CH = CH), 7.1-7.8 (m, ϕ)

. $^{19}$F-NMR (δppm, $D_2O$): -68.1

. IR (νcm$^{-1}$): 2620-3400, 1730-1770 (COOH), 1270 ($CF_3$)

. FAB-MS (M/Z): 664 ($MH^+$)

<Synthesis of complex>

The reaction was performed following the same procedures as for Reaction 7 of Example 1 by using the above ligand compound. The yield was 97%. 12.7 g of the end product were obtained. The yield of the overall process was 11%.

Example 18

Synthesis of gadolinium complex (compound-18) with N'-[3,5-bis(trifluoromethyl)benzenesulfonyl] diethylenetriamine-N,N,N",N"-tetraacetic acid

The chemical structure of the compound-18 was as follows:

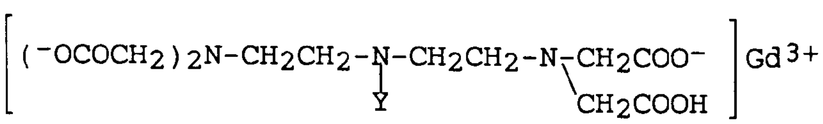

wherein

Y = 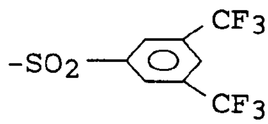

<Synthesis of ligand>

[Reaction 1]: 100 g (951 mmols) of diethanolamine and 65.7 g (475 mmols) of potassium carbonate were dissolved in 200 mℓ of distilled water. 249 g (795 mmols) of 3,5-bis(trifluoromethyl)benzenesulfonyl chloride dissolved in 300 mℓ of tetrahydrofuran were added dropwise into the resultant solution under cooling over 30 minutes. After the solution was reacted at room temperature for five hours, the organic layer was removed, and the remaining aqueous layer was extracted twice with 200 mℓ of ether. The yield was 89%.

Subsequently, Reactions 2 to 6 below were performed in this order to obtain a ligand compound.

[Reaction 2]: The same procedures as for Reaction 2 in Example 1. The yield was 84%.
[Reaction 3]: The same procedures as for Reaction 3 in Example 1. The yield was 89%.
[Reaction 4]: The same procedures as for Reaction 4 in Example 1. The yield was 88%.
[Reaction 5]: The same procedures as for Reaction 5 in Example 1. The yield was 79%.
[Reaction 6]: The same procedures as for Reaction 6 in Example 1. The yield was 87%.

The physico-chemical data of the ligand compound obtained as described above was as follows.

. Molecular formula: $C_{20}H_{23}N_3O_{10}F_6S$
. $^1H$-NMR (δppm, $D_2O$):
 2.8 (m, $CH_2N$), 3.8 (s, $CH_2CO$), 7.9-8.2 (m, ϕ)
. $^{19}F$-NMR (δppm, $D_2O$): -63.8
. IR ($\nu cm^{-1}$):
 2600-3400, 1720-1760 (COOH), 1350 ($SO_2N$), 1240 ($CF_3$)
. FAB-MS (M/Z): 612 ($MH^+$)

<Synthesis of complex>

The reaction was performed following the same procedures as for Reaction 7 of Example 1 by using the above ligand compound. The yield was 97%. 12.0 g of the end product were obtained. The yield of the overall process was 39%.

Example 19

Synthesis of gadolinium complex (compound-19) with N'-[3,5-bis(trifluoromethyl)benzenecarbonyl] diethylenetriamine-N,N,N",N"-tetraacetic acid

Chemical structure of the compound-19:

The same as the compound-18;
except that

Y = 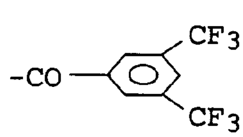

<Synthesis of ligand>

Reactions 1 to 6 were performed following the same procedures as in Example 18 except that 220 g (795 mmols) of 3,5-bis(trifluoromethyl)benzoic chloride were used in place of 249 g (795 mmols) of 3,5-bis(trifluoromethyl)benzenesulfonyl chloride in Reaction 1, thereby obtaining a ligand compound. The yields in Reactions 1 to 6 were as follows.

[Reaction 1] = 81%; [Reaction 2] = 87%;
[Reaction 3] = 88%; [Reaction 4] = 86%;
[Reaction 5] = 84%; [Reaction 6] = 84%

The physico-chemical data of the ligand compound obtained as described above was as follows.

. Molecular formula: $C_{21}H_{23}N_3O_9F_6$
. $^1$H-NMR (δppm, $D_2O$):
2.9 (m, $CH_2N$), 3.8 (s, $CH_2CO$), 7.9-8.2 (m, ϕ)
. $^{19}$F-NMR (δppm, $D_2O$): -64.9
. IR (νcm$^{-1}$):
2600-3400, 1720-1760 (COOH, CONH), 1260 ($CF_3$)
. FAB-MS (M/Z): 576 ($MH^+$)

<Synthesis of complex>

The reaction was performed following the same procedures as for Reaction 7 of Example 1 by using the above ligand compound. The yield was 95%. 12.8 g of the end product were obtained. The yield of the overall process was 36%.

Example 20

Synthesis of gadolinium complex (compound-20) with N'-[2-[3,5-bis(trifluoromethyl)phenylsulfonyl amino]ethyl]diethylenetriamine-N,N,N",N"-tetraacetic acid

The chemical structure of the compound-20 was as follows:

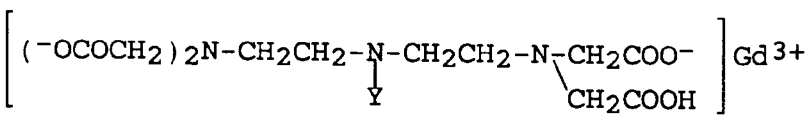

wherein

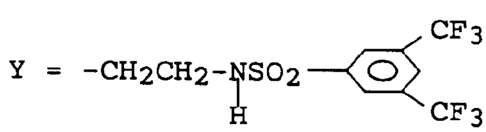

<Synthesis of ligand>

[Reaction 1]: 183 g (3.0 mols) of monoethanolamine were dissolved in 3 ℓ of tetrahydrofuran, and 2 ℓ of water in which 212 g (2.0 mols) of sodium carbonate were dissolved were added to the solution. 625 g (2.0 mols) of 3,5-bis(trifluoromethyl)sulfonyl chloride were added dropswise into the resultant solution. After the solution was reacted at room temperature for 15 hours, 2 ℓ of ethyl acetate were added to the resultant solution. The organic layer was separated, washed twice with distilled water and once with saturated brine, dried over anhydrous sodium sulfate, and concentrated under reduced pressure. The yield was 87%.

[Reaction 2]: 573 g (1.7 mols) of the product of Reaction 1 were dissolved in 1 ℓ of dry pyridine, and 398 g (2.1 mols) of p-toluenesulfonyl chloride were added to the solution under ice cooling over one hour. The resultant solution was further reacted at room temperature for one hour. The reaction mixture was poured into ice water, and the resultant solution was extracted with 2 ℓ of ethyl acetate. The organic layer was washed twice with dilute hydrochloric acid, twice with distilled water, and once with saturated brine, dried over anhydrous sodium sulfate, and concentrated under reduced pressure. The yield was 71%.

[Reaction 3]: 125 g (1.2 mols) of diethanolamine were dissolved in 1 ℓ of dry dimethylformamide, then 199 g (1.4 mols) of potassium carbonate and 590 g (1.2 mols) of the product of Reaction 2 were added to the solution. The resultant solution was reacted at 70°C for five hours. The reaction mixture was concentrated and added with distilled water, and the resultant solution was extracted with ethyl acetate. The organic layer was washed with distilled water and saturated brine, then dried over anhydrous sodium sulfate. The dried organic layer was concentrated under reduced pressure and purified through silica gel column chromatography using a solvent mixture of ethyl acetate/n-hexane = 3/7 as an eluting solution. The yield was 77%.

Subsequently, Reactions 4 to 8 below were performed in this order to obtain a ligand compound.

[Reaction 4]: The same procedures as for Reaction 2 in Example 1. The yield was 73%.

[Reaction 5]: The same procedures as for Reaction 3 in Example 1. The yield was 92%.

[Reaction 6]: The same procedures as for Reaction 4 in Example 1. The yield was 82%.
[Reaction 7]: The same procedures as for Reaction 5 in Example 1. The yield was 73%.
[Reaction 8]: The same procedures as for Reaction 6 in Example 1. The yield was 80%.
The phisico-chemical data of the ligand compound obtained as described above was as follows.

. Molecular formula: $C_{22}H_{28}N_4O_{10}SF_6$
. $^1$H-NMR (δppm, $D_2O$):
2.8 (m, $CH_2N$), 3.5 (s, $CH_2CO$), 7.7-7.8 (m, ϕ)
. $^{19}$F-NMR (δppm, D O): -68.3
. IR (νcm$^{-1}$):
2620-3410, 1730-1760 (COOH), 1340 ($SO_2N$), 1280 ($CF_3$)
. FAB-MS (M/Z): 655 ($MH^+$)

<Synthesis of complex>

The reaction was performed following the same procedures as for Reaction 7 of Example 1 by using the above ligand compound. The yield was 98%. 12.9 g of the end product were obtained. The yield of the overall process was 16%.

Example 21

Synthesis of gadolinium complex (compound-21) with N'-[2-[3,5-bis(trifluoromethyl)benzoylamino]ethyl] diethylenetriamine-N,N,N",N"-tetraacetic acid
Chemical structure of the compound-21:
The same as the compound-20;
except that

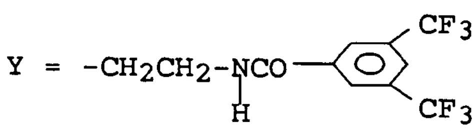

<Synthesis of ligand>

Reactions 1 to 8 were performed following the same procedures as in Example 20 except that 625 g (2.0 mols) of 3,5-bis(trifluoromethyl)benzoic chloride were used in place of 625 g (2.0 mols) of 3,5-bis(trifluoromethyl)sulfonyl chloride in Reaction 1, thereby obtaining a ligand compound. The yields in Reactions 1 to 8 were as follows.

[Reaction 1] = 87%; [Reaction 2] = 72%;
[Reaction 3] = 76%; [Reaction 4] = 74%;
[Reaction 5] = 90%; [Reaction 6] = 81%;
[Reaction 7] = 76%; [Reaction 8] = 73%

The physico-chemical data of the ligand compound obtained as described above was as follows.

. Molecular formula: $C_{23}H_{28}N_4O_9F_6$
. $^1$H-NMR (δppm, $D_2O$):
2.8 (m, $CH_2N$), 3.5 (s, $CH_2CO$), 7.6-7.8 (m, ϕ)
. $^{19}$F-NMR (δppm, D O): -68.0
. IR (νcm$^{-1}$):
2580-3400, 1740-1760 (CONH), 1260 ($CF_3$)
. FAB-MS (M/Z): 619 ($MH^+$)

<Synthesis of complex>

The reaction was performed following the same procedures as for Reaction 7 of Example 1 by using the above ligand compound. The yield was 95%. 12.7 g of the end product were obtained. The yield of the overall process was 13%.

Example 22

Synthesis of gadolinium complex (compound-22) of N'-[2-[3,5-bis(trifluoromethyl)phenylamino sulfonyl]ethyl]diethylenetriamine-N,N,N",N"-tetraacetic acid

The chemical structure of the compound-22 was as follows:

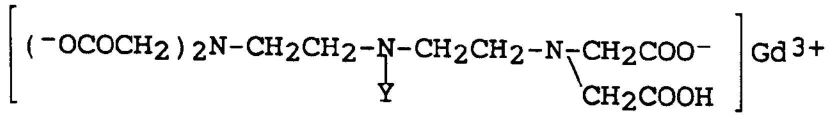

wherein

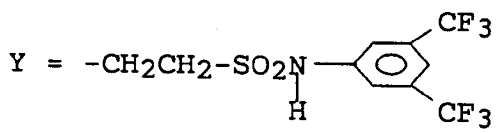

<Synthesis of ligand>

[Reaction 1]: 458 g (2.0 mols) of 3,5-bis(trifluoromethyl)aniline were dissolved in 3 ℓ of tetrahydrofuran, and 243 g (2.4 mols) of triethylamine were added to the solution. 456 g (2.2 mols) of 2-bromoethanesulfonyl chloride were added dropwise into the resultant solution, and the solution was reacted at room temperature for five hours. The reaction mixture was poured into ice water, and the resultant solution was extracted with 3 ℓ of ethyl acetate. The organic layer was washed with distilled water and saturated brine, dried over anhydrous sodium sulfate, and concentrated under reduced pressure. The yield was 67%.

Subsequently, Reactions 2 to 7 below were performed in this order to obtain a ligand compound.

[Reaction 2]: The same procedures as for Reaction 3 in Example 20. The yield was 80%.

[Reaction 3]: The same procedures as for Reaction 2 in Example 1. The yield was 70%.

[Reaction 4]: The same procedures as for Reaction 3 in Example 1. The yield was 95%.

[Reaction 5]: The same procedures as for Reaction 4 in Example 1. The yield was 80%.

[Reaction 6]: The same procedures as for Reaction 5 in Example 1. The yield was 72%.

[Reaction 7]: The same procedures as for Reaction 6 in Example 1. The yield was 79%.

The physico-chemical data of the ligand compound obtained as described above was as follows.

. Molecular formula: $C_{22}H_{28}N_4O_{10}SF_6$

. $^1$H-NMR (δppm, $D_2O$):

2.8 (m, $CH_2N$), 3.2 (t, $CH_2SO_2$), 3.5 (s, $CH_2CO$), 7.5-7.7 (m, ϕ)

. $^{19}$F-NMR (δppm, $D_2O$): -68.2

. IR (νcm$^{-1}$):

2650-3430, 1730-1770 (COOH), 1350 ($SO_2N$), 1230 ($CF_3$)

. FAB-MS (M/Z): 655 ($MH^+$)

<Synthesis of complex>

The reaction was performed following the same procedures as for Reaction 7 of Example 1 by using the above ligand compound. The yield was 98%. 13.0 g of the end product were obtained. The yield of the overall process was 16%.

Example 23

Synthesis of gadolinium complex (compound-23) of N'-[2-[3,5-bis(trifluoromethyl)phenylamino carbonyl]ethyl]diethylenetriamine-N,N,N",N"-tetraacetic acid

Chemical structure of the compound-23:

The same as the compound-22;

except that

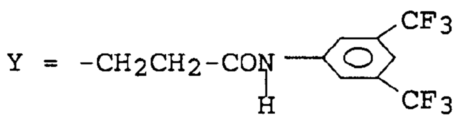

<Synthesis of ligand>

Reactions 1 to 7 were performed following the same procedures as in Example 22 except that 377 g (2.2 mols) of 3-bromopropionic chloride were used in place of 456 g (2.2 mols) of 2-bromoethanesulfonyl chloride in Reaction 1, thereby obtaining a ligand compound. The yields in Reactions 1 to 7 were as follows.

[Reaction 1] = 63%; [Reaction 2] = 83%;
[Reaction 3] = 72%; [Reaction 4] = 90%;
[Reaction 5] = 77%; [Reaction 6] = 76%;
[Reaction 7] = 82%

The physico-chemical data of the ligand compound obtained as described above was as follows.

. Molecular formula: $C_{23}H_{28}N_4O_9F_6$
. $^1$H-NMR (δppm, $D_2O$):
2.7 (m, $CH_2N$), 3.5 (s, $CH_2CO$), 7.7-7.9 (m, ϕ)
. $^{19}$F-NMR (δppm, $D_2O$): -68.3
. IR (νcm$^{-1}$):
2620-3440, 1730-1760 (CONH), 1240 ($CF_3$)
. FAB-MS (M/Z): 619 ($MH^+$)

<Synthesis of complex>

The reaction was performed following the same procedures as for Reaction 7 of Example 1 by using the above ligand compound. The yield was 96%. 12.8 g of the end product were obtained. The yield of the overall process was 17%.

Example 24

Synthesis of gadolinium complex (compound-24) with N'-[2-[3,5-bis(trifluoromethyl)phenyloxy]ethyl] diethylenetriamine-N,N,N",N"-tetraacetic acid

The chemical structure of the compound-24 was as follows:

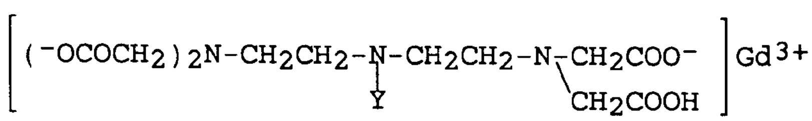

wherein

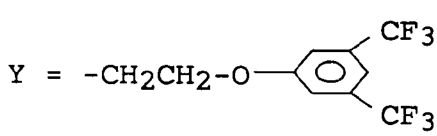

<Synthesis of ligand>

[Reaction 1]: 460 g (2.0 mols) of 3,5-bistrifluoromethyl)phenol were dissolved in 3 ℓ of dimethylformamide, and 53 g (2.2 mols) of sodium hydride were added to the solution under ice cooling. The resultant solution was stirred at room temperature for 30 minutes. 1,127 g (6.0 mols) of ethylene dibromide were added to the solution at once, and the resultant solution was reacted at 80°C for two hours. The reaction mixture was concentrated to 1/4 under reduced pressure and poured into ice water, and the resultant solution was extracted with ethyl acetate. The organic layer was washed with distilled water and saturated brine, dried over anhydrous sodium sulfate, and purified through silica gel column chromatography using a solvent mixture of ethyl acetate/n-hexane = 1/9 as an eluting solution. The yield was 72%.

Subsequently, Reactions 2 to 7 below were performed in this order to obtain a ligand compound.

[Reaction 2]: The same procedures as for Reaction 3 in Example 20. The yield was 83%.

[Reaction 3]: The same procedures as for Reaction 2 in Example 1. The yield was 72%.

[Reaction 4]: The same procedures as for Reaction 3 in Example 1. The yield was 93%.

[Reaction 5]: The same procedures as for Reaction 4 in Example 1. The yield was 80%.

[Reaction 6]: The same procedures as for Reaction 5 in Example 1. The yield was 72%.

[Reaction 7]: The same procedures as for Reaction 6 in Example 1. The yield was 78%.

The physico-chemical data of the ligand compound obtained as described above was as follows.

. Molecular formula: $C_{22}H_{27}N_3O_9F_6$

. $^1$H-NMR (δppm, $D_2O$):

2.8 (m, $CH_2N$), 3.5 (s, $CH_2CO$), 3.9 (m, $CH_2O$), 7.5-7.9 (m, ϕ)

. $^{19}$F-NMR (δppm, $D_2O$): -68.4

. IR (νcm$^{-1}$):

2660-3430, 1730-1780 (CONH), 1290 ($CF_3$), 1240 (-O-)

. FAB-MS (M/Z): 592 ($MH^+$)

<Synthesis of complex>

The reaction was performed following the same procedures as for Reaction 7 of Example 1 by using the above ligand compound. The yield was 93%. 12.5 g of the end product were obtained. The yield of the overall process was 18%.

Example 25

Synthesis of gadolinium complex (compound-25) with N'-[4-[3,5-bis(trifluoromethyl)phenyloxy]-n-butyl]diethylenetriamine-N,N,N",N"-tetraacetic acid

Chemical structure of the compound-25:

The same as the compound-24;

except that

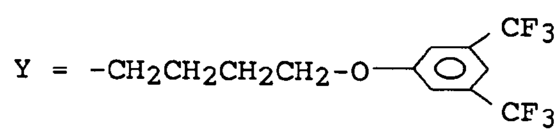

<Synthesis of ligand>

Reactions 1 to 7 were performed following the same procedures as in Example 24 except that 1,296 g (6.0 mols) of 1,4-dibromobutane were used in place of 1,127 g (6.0 mols) of ethylene dibromide in Reaction 1, thereby obtaining a ligand compound. The yields in Reactions 1 to 7 were as follows.

[Reaction 1] = 74%; [Reaction 2] = 85%;

[Reaction 3] = 77%; [Reaction 4] = 90%;

[Reaction 5] = 78%; [Reaction 6] = 73%;

[Reaction 7] = 76%

The physico-chemical data of the ligand compound obtained as described above was as follows.

. Molecular formula: $C_{24}H_{31}N_3O_9F_6$

. $^1$H-NMR (δppm, $D_2O$):

1.7-1.9 (m, $CCH_2CH_2C$), 2.7 (m, $CH_2N$), 3.6 (s, $CH_2CO$), 3.8 (m, $CH_2O$), 7.6-7.8 (m, ϕ)

. $^{19}$F-NMR (δppm, $D_2O$): -68.1

. IR (νcm$^{-1}$):

2610-3400, 1720-1750 (COOH), 1250 (-O-), 1210 ($CF_3$)

. FAB-MS (M/Z): 620 ($MH^+$)

<Synthesis of complex>

The reaction was performed following the same procedures as for Reaction 7 of Example 1 by using the

above ligand compound. The yield was 96%. 12.8 g of the end product were obtained. The yield of the overall process was 19%.

Example 26

Synthesis of gadolinium complex (compound-26) with N'-[2-[3,5-bis(trifluoromethyl)phenylthio]ethyl] diethylenetriamine-N,N,N",N"-tetraacetic acid

The chemical structure of the compound-26 was as follows:

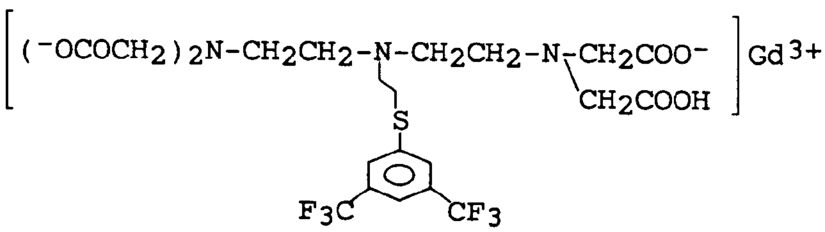

<Synthesis of ligand>

[Reaction 1]: 492 g (2.0 mols) of 3,5-bis(trifluoromethyl)thiophenol were dissolved in 3 ℓ of dimethylformamide, and 53 g (2.2 mols) of sodium hydride were added to the solution under ice cooling. The resultant solution was stirred at room temperature for 30 minutes. 1,127 g (6.0 mols) of ethylene dibromide were added to the solution at once, and the resultant solution was reacted at 80°C for two hours. The reaction mixture was concentrated to 1/4 under reduced pressure and poured into ice water, and the resultant solution was extracted with ethyl acetate. The organic layer was washed with distilled water and saturated brine, dried over anhydrous sodium sulfate, and purified through silica gel column chromatography using a solvent mixture of ethyl acetate/n-hexane = 1/9 as an eluting solution. The yield was 68%.

Subsequently, Reactions 2 to 7 below were performed in this order to obtain a ligand compound.

[Reaction 2]: The same procedures as for Reaction 3 in Example 20. The yield was 81%.

[Reaction 3]: The same procedures as for Reaction 2 in Example 1. The yield was 69%.

[Reaction 4]: The same procedures as for Reaction 3 in Example 1. The yield was 91%.

[Reaction 5]: The same procedures as for Reaction 4 in Example 1. The yield was 86%.

[Reaction 6]: The same procedures as for Reaction 5 in Example 1. The yield was 75%.

[Reaction 7]: The same procedures as for Reaction 6 in Example 1. The yield was 80%.

The physico-chemical data of the ligand compound obtained as described above was as follows.

. Molecular formula: $C_{22}H_{27}N_3O_8SF_6$

. $^1$H-NMR (δppm, $D_2O$):

2.4 (t, $CH_2S$), 2.8 (m, $CH_2N$), 3.5 (s, $CH_2CO$), 7.3-7.5 (m, ϕ)

. $^{19}$F-NMR (δppm, $D_2O$): -68.0

. IR (νcm$^{-1}$):

2600-3420, 1720-1740 (COOH), 1250 ($CF_3$)

. FAB-MS (M/Z): 608 ($MH^+$)

<Synthesis of complex>

The reaction was performed following the same procedures as for Reaction 7 of Example 1 by using the above ligand compound. The yield was 90%. 12.1 g of the end product were obtained. The yield of the overall process was 15%.

Example 27

Synthesis of gadolinium complex (compound-27) with N'-[N''',N'''-bis[3,5-bis(trifluoromethyl)benzyl] aminoethyl]diethylenetriamine-N,N,N",N"-tetraacetic acid

The chemical structure of the compound-27 was as follows:

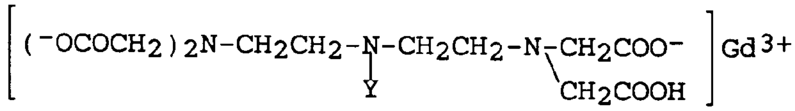

wherein

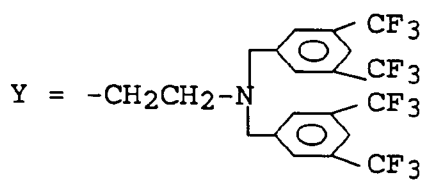

<Synthesis of ligand>

[Reaction 1]: 122 g (2.0 mols) of ethanolamine were dissolved in 3 ℓ of dry tetrahydrofuran, and 405 g (4.0 mols) of triethylamine and 1,228 g (4.0 mols) of 3,5-bis(trifluoromethyl)benzyl bromide were added to the solution. The resultant solution was refluxed under heating for three hours. After the reaction, the reaction mixture was concentrated under reduced pressure and added with 3 ℓ of ethyl acetate. The resultant solution was washed twice with distilled water and once with saturated brine, dried over anhydrous sodium sulfate, and purified through silica gel column chromatography using a solvent mixture of ethyl acetate/n-hexane = 3/7 as an eluting solution. The yield was 81%.

[Reaction 2]: 832 g (1.6 mols) of the product of Reaction 1 were dissolved in 1 ℓ of dry pyridine, and 336 g (1.8 mols) of p-toluenesulfonyl chloride were added to the solution under ice cooling over one hour. The resultant solution was reacted at room temperature for one hour. The reaction mixture was poured into ice water, and the resultant solution was extracted with 2 ℓ of ethyl acetate. The organic layer was washed twice with dilute hydrochloric acid, twice with distilled water, and once with saturated brine, dried over anhydrous sodium sulfate, and concentrated under reduced pressure. The yield was 88%.

Subsequently, Reactions 3 to 8 below were performed in this order to obtain a ligand compound.

[Reaction 3]: The same procedures as for Reaction 3 in Example 20. The yield was 75%.

[Reaction 4]: The same procedures as for Reaction 2 in Example 1. The yield was 70%.

[Reaction 5]: The same procedures as for Reaction 3 in Example 1. The yield was 90%.

[Reaction 6]: The same procedures as for Reaction 4 in Example 1. The yield was 80%.

[Reaction 7]: The same procedures as for Reaction 5 in Example 1. The yield was 80%.

[Reaction 8]: The same procedures as for Reaction 6 in Example 1. The yield was 82%.

The physico-chemical data of the ligand compound obtained as described above was as follows.

. Molecular formula: $C_{32}H_{34}N_4O_8F_{12}$

. $^1$H-NMR (δppm, $D_2O$):

2.9 (m, $CH_2N$), 3.4 (s, $CH_2CO$), 7.7-7.9 (m, ϕ)

. $^{19}$F-NMR (δppm, $D_2O$): -68.3 ($CF_3$)

. IR (νcm$^{-1}$):

2600-3410, 1740-1760 (COOH), 1210 ($CF_3$)

. FAB-MS (M/Z): 831 ($MH^+$)

<Synthesis of complex>

The reaction was performed following the same procedures as for Reaction 7 of Example 1 by using the above ligand compound. The yield was 97%. 12.0 g of the end product were obtained. The yield of the overall process was 18%.

Example 28

Synthesis of gadolinium complex (compound-28) with N'-[N'''-[3,5-bis(trifluoromethyl)benzyl] aminoethyl]diethylenetriamine-N,N,N",N"-tetraacetic acid

Chemical structure of the compound-28:

The same as the compound-27;

except that

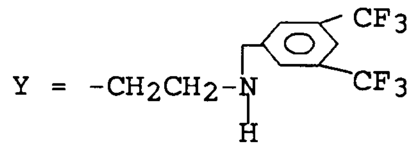

<Synthesis of ligand>

[Reaction 1]: The reaction was performed following the same procedures as for Reaction 1 in Example 27 except that the addition amount of 3,5-bis(trifluoromethyl)benzyl bromide was changed to 614 g (2.0 mols) and the reaction was performed at room temperature for three hours. In addition, a solvent mixture of ethyl acetate/n-hexane = 4/6 was used as an eluting solution in silica gel column chromatography. The yield was 73%.

[Reaction 2]: 534 g (1.9 mols) of the product of Reaction 1 were dissolved in 5 ℓ of chloroform, and 226 g (2.2 mols) of triethylamine and 482 g (2.2 mols) of o-nitrobenzyl bromide were added to the solution. The resultant solution was refluxed under heating for four hours. The reaction mixture was concentrated under reduced pressure and added with 3 ℓ of ethyl acetate. The ethyl acetate solution was washed twice with distilled water and once with saturated brine, dried over anhydrous sodium sulfate, and purified through silica gel column chromatography using a solvent mixture of ethyl acetate/n-hexane = 3/7 as an eluting solution. The yield was 90%.

Subsequently, Reactions 3 to 9 below were performed in this order to obtain a ligand compound.

[Reaction 3]: The same procedures as for Reaction 2 in Example 27. The yield was 87%.

[Reaction 4]: The same procedures as for Reaction 3 in Example 20. The yield was 73%.

[Reaction 5]: The same procedures as for Reaction 2 in Example 1. The yield was 70%.

[Reaction 6]: The same procedures as for Reaction 3 in Example 1. The yield was 93%.

[Reaction 7]: The same procedures as for Reaction 4 in Example 1. The yield was 82%.

[Reaction 8]: The same procedures as for Reaction 5 in Example 1. The yield was 81%.

[Reaction 9]: The same procedures as for Reaction 6 in Example 1. The yield was 85%.

The physico-chemical data of the ligand compound obtained as described above was as follows.

. Molecular formula: $C_{30}H_{36}N_5O_{10}F_6$

. $^1$H-NMR (δppm, $D_2O$):

2.7 (m, $CH_2N$), 3.3 (s, $CH_2CO$), 7.7-7.9 (m, ϕ)

. $^{19}$F-NMR (δppm, $D_2O$): -67.8

. IR (νcm$^{-1}$):

2610-3430, 1740-1780 (COOH), 1230 ($CF_3$)

. FAB-MS (M/Z): 740 ($MH^+$)

<Synthesis of complex>

[Reaction 10]: The reaction was performed following the same procedures as for Reaction 7 of Example 1 by using the above ligand compound as a starting material. The yield was 98%.

[Reaction 11]: 12.5 g (13 mmols) of the product of Reaction 10 were dissolved in 500 mℓ of methanol, and 1.0 g of 10% palladium carbon was added to the solution, thereby performing a catalytic hydrogenation reaction at normal pressure. After the solution was reacted for five hours, the catalyst was filtered off, and the filtrate was concentrated to about 1/3 under reduced pressure. Isopropanol was added to the concentrated solution, and the precipitate formed was filtered out, washed with a small amount of isopropanol, and dried. The yield was 96%. 10.3 g of the end product were obtained. The yield of the overall process was 15%.

Example 29

Synthesis of gadolinium complex (compound-29) with N'-[N'''-[3,5-bis(trifluoromethyl)benzyl] aminoethyl]diethylenetriamine-N,N,N'',N'',N'''-pentaacetic acid

Chemical structure of the compound-29:

The same as the compound-27;

except that

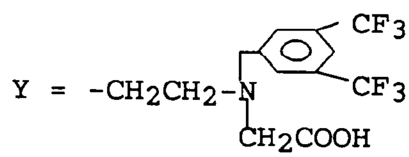

<Synthesis of ligand>

[Reaction 1]: 534 g (1.9 mols) of the product of Reaction 1 in Example 28 were dissolved in 5 ℓ of chloroform, and 226 g (2.2 mols) of triethylamine and 367 g (2.2 mols) of ethyl bromoacetate were added to the solution. The resultant solution was reacted at room temperature for two hours. The reaction mixture was poured into ice water, and the resultant solution was extracted with ethyl acetate. The extract was washed with distilled water and saturated brine, dried over anhydrous sodium sulfate, and purified through silica gel column chromatography using a solvent mixture of ethyl acetate/n-hexane = 3/7 as an eluting solution. The yield was 85%.

Subsequently, Reactions 2 to 8 below were performed in this order to obtain a ligand compound.

[Reaction 2]: The same procedures as for Reaction 2 in Example 27. The yield was 86%.
[Reaction 3]: The same procedures as for Reaction 3 in Example 20. The yield was 74%.
[Reaction 4]: The same procedures as for Reaction 2 in Example 1. The yield was 72%.
[Reaction 5]: The same procedures as for Reaction 3 in Example 1. The yield was 90%.
[Reaction 6]: The same procedures as for Reaction 4 in Example 1. The yield was 83%.
[Reaction 7]: The same procedures as for Reaction 5 in Example 1. The yield was 82%.
[Reaction 8]: The same procedures as for Reaction 6 in Example 1. The yield was 89%.

The physico-chemical data of the ligand compound obtained as described above was as follows.

. Molecular formula: $C_{25}H_{32}N_4O_{10}F_6$
. $^1$H-NMR (δppm, $D_2O$):
  2.7 (m, $CH_2N$), 3.6 (s, $CH_2CO$), 7.4-7.7 (m, ϕ)
. $^{19}$F-NMR (δppm, $D_2O$): -68.4
. IR (νcm$^{-1}$):
  2600-3450, 1730-1760 (COOH), 1240 ($CF_3$)
. FAB-MS (M/Z): 663 ($MH^+$)

<Synthesis of complex>

The reaction was performed following the same procedures as for Reaction 7 of Example 1 by using the above ligand compound as a starting material. The yield was 95%. 12.4 g of the end product were obtained. The yield of the overall process was 19%.

Example 30

Synthesis of copper complex (compound-30) with N'-[3,5-bis(trifluoromethyl)benzyl] diethylenetriamine-N,N,N",N"-tetraacetic acid

The chemical structure of the compound-30 was as follows:

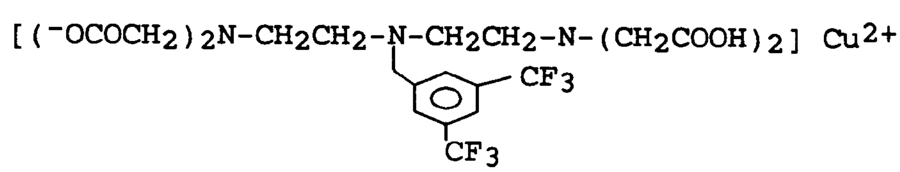

10 g (18 mmols) of the ligand compound obtained by Reaction 6 of Example 8 were dissolved in 100 mℓ of distilled water containing 36 mℓ of 1 N sodium hydroxide, and 2.9 g (36 mmols) of cupric oxide were added to the solution. The resultant solution was reacted at 80°C for three hours. The reaction mixture was filtered to remove unreacted cupric oxide. The filtrate was treated by a cation exchange resin (Amberlite IR-120B) and evaporated to dryness under reduced pressure, thereby obtaining the objective product. The yield was 98%. 11.2 g of the end product were obtained. The yield of the overall process was 41%.

Example 31

Synthesis of chromium complex (compound-31) of N'-[3,5-bis(trifluoromethyl)benzyl] diethylenetriamine-N,N,N",N"-tetraacetic acid

The chemical structure of the compound-31 was as follows:

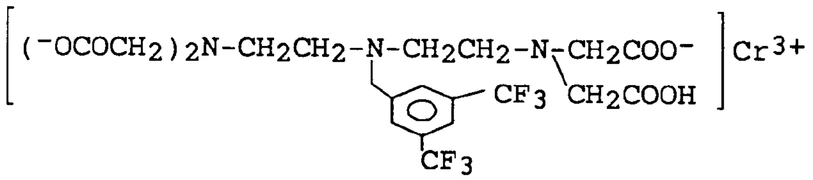

The objective product was obtained following the same procedures as in Example 30 except that 2.7 g (18 mmols) of chromium(III) oxide were used in place of 2.9 g (36 mmols) of copric oxide. The yield was 96%. 11.4 g of the end product were obtained. The yield of the overall process was 40%.

Example 32

Synthesis of manganese complex (compound-32) of N'-[3,5-bis(trifluoromethyl)benzyl] diethylenetriamine-N,N,N",N"-tetraacetic acid

The chemical structure of the compound-32 was as follows:

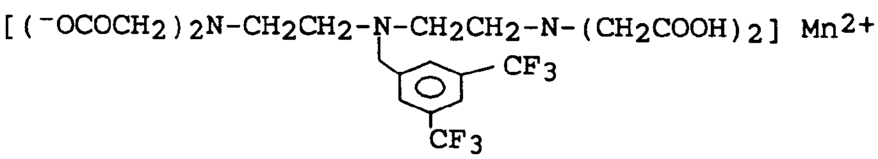

The objective product was obtained following the same procedures as in Example 30 except that 3.1 g (36 mmols) of manganese dioxide were used in place of 2.9 g (36 mmols) of copric oxide. The yield was 97%. 11.5 g of the end product were obtained. The yield of the overall process was 41%.

Example 33 (measurement of relaxation time)

Each metal complex compound of the present invention was prepared into a 10 mM aqueous solution, and the $T_1$ and $T_2$ values of intramolecular fluorine atoms contained in the solution were measured by using 90 MHz-NMR (EX-90 available from JEOL). The results are summarized in Table 1 below.

Table 1

| Compound | T1 value | T2 value |
|---|---|---|
| Compound-1 | 1.63 | 1.57 |
| Compound-5 | 1.71 | 1.70 |
| Compound-8 | 2.39 | 2.37 |
| Compound-9 | 2.58 | 2.54 |
| Compound-11 | 2.37 | 2.40 |
| Compound-16 | 2.41 | 2.32 |
| Compound-17 | 12.3 | 7.55 |
| Compound-18 | 2.79 | 2.63 |
| Compound-20 | 3.81 | 3.74 |
| Compound-25 | 4.52 | 4.48 |
| Compound-30 | 31.5 | 26.4 |
| Compound-31 | 15.3 | 13.1 |
| (unit:msec, measurement concentration:10mM) | | |

Example 34

Measurement of imaging sensitivity (phantom experiment)

Each of the aqueous metal complex compound solutions with various concentrations of the present invention was sealed in a cylindrical acryl phantom with an inner diameter of 1 cm and a height of 10 cm and subjected to imaging. SIGNA (1.5T) available from GE was used as an MRI apparatus, and imaging was performed by using a surface coil 13 cm in diameter for fluorine. A GRASS method was used as a pulse sequence, and the echo time (TE), the repetition time (TR), the flip angle, the slice width, and the matrix were set at 2 msec, 10 msec, 90°, 2.0 cm, and 256 × 128, respectively. The measurement was performed for 20 minutes, and the number of integration times was 937. As references, alkaline aqueous solutions of 5-fluorouracil with various concentrations were imaged by an identical apparatus. Conditions for obtaining the highest imaging sensitivity were examined and were found to be a spin echo method as the pulse sequence, a TE of 100 msec, and a TR of 4.5 sec. The imaging time was 20 minutes, and the number of integration times was twice.

The results are shown in Table 2 below. As can be seen from Table 2, it was impossible to detect the aqueous 5-fluorouracil solution as a clear image unless the concentration of the solution was 500 mM or more. In contrast, each metal complex compound of the present invention could be detected clearly at a concentration of a minimum of 500 μM to 5 mM. Other compounds of the present invention not recited in Table 2 also exhibited substantially the same sensitivities.

Table 2

| Compound | Concentration 500 mM | 100 mM | 50 mM | 10 mM | 5 mM | 1 mM | 500 μM | 100 μM |
|---|---|---|---|---|---|---|---|---|
| 5-FU | ○ | △ | △ | × | × | × | × | × |
| Compound-8 | ○ | ○ | ○ | ○ | ○ | ○ | △ | × |
| Compound-9 | ○ | ○ | ○ | ○ | ○ | ○ | △ | × |
| Compound-17 | ○ | ○ | ○ | ○ | ○ | ○ | △ | × |
| Compound-27 | ○ | ○ | ○ | ○ | ○ | ○ | ○ | △ |
| Compound-30 | ○ | ○ | ○ | ○ | ○ | △ | × | × |

(5-FU : 5-fluorouracil as reference sample)

○ : detectable as clear image
△ : marginally detectable as image
× : undetectable

Example 35

Metal complex compound labeling reaction of monoclonal antibody

7.0 mg (10.3 μmols) of the compound-28 were dissolved in a sodium carbonate buffer solution (pH 8.0), and 2.5 mg (13.1 μmols) of N-(3-dimethylaminopropyl)-N'-ethylcarbodiimide were added to the solution at 0°C. Subsequently, 2 mg of an anti-CEA monoclonal antibody (mouse IgGl available from Japan Biotest Laboratory) dissolved in 1 mℓ of PBS were added to the resultant solution. The solution was further reacted at room temperature four two hours. Excess reagent and unreacted components were removed by dialysis using a phosphoric acid buffer solution and by performing centrifugal separation several times. The resultant preparation was purified through a sephadex G-100 column and freeze-dried. A labeling ratio was calculated from a Gd quantity measured by inductively coupled plasma emission spectroscopy and a protein quantity determined by a Lowry process. The result was a composite in which 12.7 molecules of the compound-28 were bonded to each antibody on average.

Example 36

Imaging of tumor tissue using mouse

The compound-28 coupled with the anti-CEA antibody which was prepared in Example 35 was dissolved in physiological saline added with a small amount of albumin, and the solution was adjusted to have a Gd concentration of 1 mM. 2.0 mℓ of the resultant solution were intravenously injected into a nude mouse to which a human carcinoma of the colon and rectum was transplanted. 24 hours after the injection, MRI imaging was performed under the same imaging conditions as in Example 34.

As a control experiment, a similar anti-CEA antibody which was $^{131}I$-labeled by a chloramine T method was given to a mouse in the same manner as described above, and imaging was performed by using a gamma camera (STARCAM available from GE) 24 hours after the injection.

As a result, the tumor tissue could be detected as a clear image by the method using the metal complex compound of the present invention, and almost no image articraft was found. In addition, the contrast with respect to the background was higher than and the image resolution was much higher than those of the control experiment performed by means of nuclear medicine.

## Claims

1. A $^{19}F$-MRI contrast medium for MRI using $^{19}F$ as a detectable nucleus, characterized by comprising a metal complex compound in which a polyamine ligand containing not less than one fluorine atom is coordinatebonded to an ion of a paramagnetic metal.

2. A contrast medium according to claim 1, characterized in that said paramagnetic metal is a member selected from the group consisting of chromium, manganese, iron, copper, and gadolinium.

3. A contrast medium according to claim 1, characterized in that said polyamine contains a plurality of fluorine atoms.

4. A contrast medium according to claim 3, characterized in that NMR chemical shifts of said plurality of fluorine atoms are essentially the same.

5. A contrast medium according to claim 4, characterized in that all of the NMR chemical shifts of said plurality of fluorine atoms are distributed within a range of not broader than 2,000 Hz.

6. A contrast medium according to claim 1, characterized in that a tissue specific substance with a specific affinity for a particular living tissue to be imaged is bonded to said polyamine ligand.

7. A contrast medium according to claim 6, characterized in that said tissue specific substance is bonded to said polyamine ligand via a suitable linker.

8. A contrast medium according to claim 6, characterized in that said tissue specific substance is a substance comprising a peptide and/or a sugar chain.

9. A contrast medium according to claim 6, characterized in that said tissue specific substance is a monoclonal antibody.

10. A contrast medium according to claim 1, characterized by further comprising assistants or additives normally used in pharmaceutics and selected from fillers, stabilizers, surfactants, buffering agents, electrolytes, coloring agents, perfumes, and flavoring agents,

    wherein said contrast medium is prepared into tablets, a powdered medicine, or a liquid medicine.

11. A contrast medium according to claim 1, characterized in that said polyamine complex compound is a member selected from the group consisting of a metal complex compound represented by the following formula and a physiologically acceptable salt thereof:

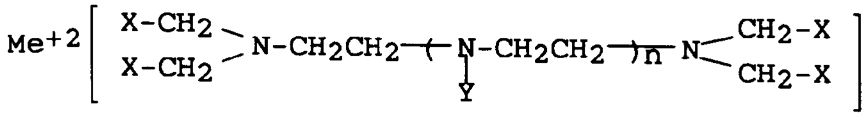

wherein Me, z, X, n, and Y have the following meanings;

Me : paramagnetic metal ion

z : an ionic valence of Me, a positive integer (preferably 2 or 3)

X : which may be the same or different and represents $-COO^-$, $-COOZ$, $-PO_3HZ$, -CONHW, or -OH wherein Z and W are;

Z : a hydrogen atom or an organic or inorganic base equivalent or metal ion equivalent

W : a straight chain or branched chain alkyl group substituted with not less than one -OH group

n : an integer from 1 to 3

Y : when n = 1, Y is R, wherein R is a substituent which has not less than one fluorine atom and may contain X defined above, and

when n = 2 or 3, at least one Y is R defined above and each of remaining Ys is $-CH_2X$, a lower alkyl group, or a hydrogen atom,

and wherein a ligand portion of the metal complex compound is coordinate-bonded to said paramagnetic metal ion so as to occupy coordinate sites via at least some of nitrogen atoms and/or oxygen atoms which are contained in said ligand portion and can be coordinated.

**12.** A contrast medium according to claim 11, characterized in that said -R is $-R_{1F}$, wherein $-R_{1F}$ means a straight chain or branched chain alkyl group which is substituted with not less than one fluorine atom and may contain X defined above.

**13.** A contrast medium according to claim 11, characterized in that said -R is a member selected from the group consisting of

$-R_2-Ar_F$, $-R_2-\Phi-R_2-Ar_F$;
$-SO_2-R_{1F}$, $-SO_2-R_2-R_{1F}$, $-SO_2-Ar_F$, $-SO_2-R_2-Ar_F$;
$-CO-R_{1F}$, $-CO-R_2-R_{1F}$, $-CO-Ar_F$, $-CO-R_2-Ar_F$;
$-R_2-NH-SO_2-R_{1F}$, $-R_2-NH-SO_2-Ar_F$, $-R_2-NH-SO_2-R_2-Ar_F$;
$-R_2-SO_2-NH-R_{1F}$, $-R_2-SO_2-NH-Ar_F$, $-R_2-SO_2-NH-R_2-Ar_F$;
$-R_2-NH-CO-R_{1F}$, $-R_2-NH-CO-Ar_F$, $-R_2-NH-CO-R_2-Ar_F$;
$-R_2-CO-NH-R_{1F}$, $-R_2-CO-NH-Ar_F$, $-R_2-CO-NH-R\ -Ar_F$;
$-R_2-S-R_{1F}$, $-R_2-S-Ar_F$, $-R_2-S-R_2-Ar_F$;
$-R_2-O-R_{1F}$, $-R_2-O-Ar_F$, $-R_2-O-R_2-Ar_F$;
$-R_2-NH-R_{1F}$, $-R_2-NH-R_2-Ar_F$;
$-R_2-N(R_3)-R_{1F}$, $-R_2-N(R_3)-R_2-Ar_F$;
$-R_2-N(X)-R_{1F}$, $-R_2-N(X)-R_2-Ar_F$;
$-R_2-N(R_{1F})-R_2-Ar_F$, and
$-R_2-N(R_2-Ar_F)_2$,

and wherein each of X and -R1F represents the same meanings as defined above, and $-R_2-$, $-R_3$, $-\Phi-$, and $-Ar_F$ have the following meanings;

$-R_2-$ :a saturated or unsaturated hydrocarbon chain

$-R_3$ :a lower alkyl group

$-\Phi-$ :a phenylene group

$-Ar_F$ :

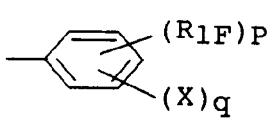

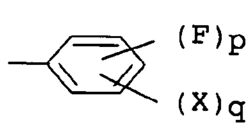

(p represents an integer from 1 to 5, and q represents 0 or an integer from 1 to 4).